(19) Europäisches Patentamt
European Patent Office
Office européen des brevets

(11) EP 4 585 059 A1

(12) **EUROPEAN PATENT APPLICATION**

(43) Date of publication:
**16.07.2025 Bulletin 2025/29**

(21) Application number: **24151724.2**

(22) Date of filing: **12.01.2024**

(51) International Patent Classification (IPC):
***A24B 13/00*** *(2006.01)* ***A24B 15/16*** *(2020.01)*
***A24B 15/28*** *(2006.01)* ***A61K 9/00*** *(2006.01)*

(52) Cooperative Patent Classification (CPC):
**A24B 15/281; A24B 13/00; A24B 15/16; A61K 9/0056; A61K 9/006**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(71) Applicant: **Nicoventures Trading Limited**
**London WC2R 3LA (GB)**

(72) Inventors:
- **Von Cosmos, Nicolas**
  **North Caroline (US)**
- **Zawadzki, Michael A**
  **North Carolina (US)**

(74) Representative: **Newcombe, Christopher David et al**
**Venner Shipley LLP**
**200 Aldersgate**
**London EC1A 4HD (GB)**

# (54) EFFERVESCENT POUCHED PRODUCT

(57) The disclosure provides a pouched product configured for oral use. The product includes a pouch and an effervescent composition enclosed therein. The effervescent composition includes an effervescent material capable of causing effervescence in the oral cavity; a filler component; and at least one active ingredient.



Processed by Luminess, 75001 PARIS (FR)

## Description

### FIELD OF THE DISCLOSURE

The present disclosure relates to oral products. In particular, the present disclosure relates to products intended for human consumption. The products are configured for oral use and deliver substances such as flavors and/or active ingredients during use.

### BACKGROUND

There are many categories of products intended for oral use and enjoyment. For example, oral tobacco products containing nicotine, which is known to have both stimulant and anxiolytic properties, have been available for many years. Conventional formats for so-called "smokeless" tobacco products include moist snuff, snus, and chewing tobacco, which are typically formed almost entirely of particulate, granular, or shredded tobacco, and which are either portioned by the user or presented to the user in individual portions, such as in single-use pouches or sachets. See for example, the types of smokeless tobacco formulations, ingredients, and processing methodologies set forth in US Pat. Nos. 6,668,839 to Williams; 6,834,654 to Williams; 6,953,040 to Atchley et al.; 7,032,601 to Atchley et al.; and 7,694,686 to Atchley et al.; 7,810,507 to Dube et al.; 7,819,124 to Strickland et al.; 7,861,728 to Holton, Jr. et al.; 7,901,512 to Quinter et al.; 8,627,828 to Strickland et al.; 11,246,334 to Atchley, each of which is incorporated herein by reference.

In addition, traditional tobacco materials and non-tobacco materials have been combined with other ingredients to form product formats distinct from traditional smokeless products, with example formats including lozenges, pastilles, gels, and the like. See, for example, the types of products described in US Patent App. Pub. Nos. 2008/0196730 to Engstrom et al.; 2008/0305216 to Crawford et al.; 2009/0293889 to Kumar et al.; 2010/0291245 to Gao et al; 2011/0139164 to Mua et al.; 2012/0037175 to Cantrell et al.; 2012/0055494 to Hunt et al.; 2012/0138073 to Cantrell et al.; 2012/0138074 to Cantrell et al.; 2013/0074855 to Holton, Jr.; 2013/0074856 to Holton, Jr.; 2013/0152953 to Mua et al.; 2013/0274296 to Jackson et al.; 2015/0068545 to Moldoveanu et al.; 2015/0101627 to Marshall et al.; and 2015/0230515 to Lampe et al., each of which is incorporated herein by reference.

There is continuing interest in the development of new types of oral products that deliver advantageous sensorial or biological activity. Such products typically contain flavorants and/or active ingredients such as nicotine, caffeine, botanicals, or cannabidiol. The format of such products can vary and include pouched products containing a powdered or granular composition, lozenges, pastilles, liquids, gels, emulsions, meltable compositions, and the like. See, for example, the types of products described in US Patent App. Pub. Nos. 2022/0160675 to Gerardi et al.; 2022/0071984 to Poole et al.; 2021/0378948 to Gerardi et al.; 2021/0330590 to Hutchens et al.; 2021/0186081 to Gerardi et al.; 2021/0177754 to Keller et al; 2021/0177043 to Gerardi et al.; 2021/0177038 to Gerardi et al.; 2021/0169867 to Holton, Jr. et al.; 2021/0169792 to Holton, Jr. et al.; 2021/0169132 to Holton, Jr. et al.; 2021/0169121 to St. Charles, and 2021/0169122 to St. Charles, each of which is incorporated herein by reference.

### BRIEF SUMMARY

The present disclosure generally provides pouched products configured for oral use. The products comprise a pouch and an effervescent composition enclosed therein. The effervescent composition generally comprises an effervescent material, a filler, and one or more active ingredients.

Accordingly, in one aspect is provided a pouched product configured for oral use comprising a pouch and an effervescent composition enclosed therein, the effervescent composition comprising: an effervescent material capable of causing effervescence in the oral cavity; a filler component; and at least one active ingredient.

In some embodiments, the effervescent material comprises a sugar material containing an entrapped gaseous component, such that release of the entrapped gaseous component occurs upon dissolution of the sugar material in the oral cavity.

In some embodiments, the effervescent material is carbonated isomalt.

In some embodiments, the effervescent material comprises: an acid component; a base component, wherein the base component is a carbonate material, a bicarbonate material, or a mixture thereof; and optionally, a binder, a humectant, or a combination thereof.

In some embodiments, the effervescent material further comprises a binder, a humectant, or a combination thereof. In some embodiments, the binder is a sugar alcohol. In some embodiments, the humectant is glycerol.

In some embodiments, the acid component is a tricarboxylic acid, a dicarboxylic acid, or a combination thereof.

In some embodiments, the acid component comprises a combination of a tricarboxylic acid and a dicarboxylic acid in a weight ratio of from about 2:1 to about 1:2.

In some embodiments, the acid component is citric acid, tartaric acid, or a combination thereof.

In some embodiments, the acid component is a combination of citric acid and tartaric acid in a ratio of from about 2:1 to about 1:2 by weight.

In some embodiments, the base component is a bicarbonate material.

In some embodiments, the acid component is present in an amount of from about 10% to about 20% by weight, based on the total dry weight of the effervescent material.

In some embodiments, the acid component and the base component are present in about a 1:1 molar ratio.

In some embodiments, the at least one active ingredient comprises one or more flavoring agents, botanical materials, stimulants, nicotine components, amino acids, vitamins, cannabinoids, cannabimimetics, terpenes, nutraceuticals, pharmaceutical agents, or combinations thereof.

In some embodiments, the at least one active ingredient comprises a nicotine component.

In some embodiments, the nicotine component is selected from the group consisting of free base nicotine, a nicotine salt, an ion-paired nicotine complex, resin bound nicotine, and combinations thereof.

In some embodiments, the effervescent composition is present in the pouch as a mixture of discrete particles of the effervescent material and the filler component.

In some embodiments, the filler component comprises a cellulosic material.

In some embodiments, the cellulosic material is cellulose or microcrystalline cellulose.

In some embodiments, the at least one active ingredient is adsorbed in or on the filler component.

In some embodiments, the cellulosic material is in the form of spheres.

In some embodiments, the effervescent material is coated with a lipid, a cellulose ether, shellac, gum arabic, or a combination thereof.

In some embodiments, the lipid is an oil selected from the group consisting of palm oil, palm kernel oil, soybean oil, cottonseed oil, and combinations thereof, wherein the oil may be hydrogenated, partially hydrogenated, or non-hydrogenated.

In some embodiments, the pouched product further comprises one or more additives selected from the group consisting of sweeteners, taste modifiers, salts, binders, plasticizers, buffering agents, colorants, humectants, oral care additives, preservatives, disintegration aids, antioxidants, flow aids, and combinations thereof.

In some embodiments, the effervescent material is present in granulated form, the effervescent material comprising isomalt, glycerin, an acid component, and a base component; and the filler component is present as cellulose spheres, the cellulose spheres comprising cellulose, a nicotine component, one or more salts, one or more sweeteners, and a flavoring agent.

In some embodiments, the isomalt is present in an amount in a range from about 38 to about 56% by weight, based on the total weight of the effervescent material, and the glycerin is present in an amount in a range 6 to 10 by weight, based on the total weight of the effervescent material.

In some embodiments, the pouched product comprises from about 40 to about 60 percent by weight of the effervescent material; and from about 40 to about 60 percent by weight of the filler component, based on the total weight of the effervescent composition.

The disclosure includes, without limitations, the following embodiments.

Embodiment 1: A pouched product configured for oral use comprising a pouch and an effervescent composition enclosed therein, the effervescent composition comprising:

an effervescent material capable of causing effervescence in the oral cavity;
a filler component; and
at least one active ingredient.

Embodiment 2: The pouched product of embodiment 1, wherein the effervescent material comprises a sugar material containing an entrapped gaseous component, such that release of the entrapped gaseous component occurs upon dissolution of the sugar material in the oral cavity.

Embodiment 3: The pouched product of embodiment 1 or 2, wherein the effervescent material is carbonated isomalt.

Embodiment 4: The pouched product of embodiment 1, wherein the effervescent material comprises an acid component and a base component, wherein the base component is a carbonate material, a bicarbonate material, or a mixture thereof.

Embodiment 5: The pouched product of any one of embodiments 1-4, wherein the effervescent material further comprises a binder, a humectant, or a combination thereof.

Embodiment 6: The pouched product of embodiment 5, wherein the binder is a sugar alcohol.

Embodiment 7: The pouched product of any one of embodiments 4-6, wherein the acid component is a tricarboxylic acid, a dicarboxylic acid, or a combination thereof.

Embodiment 8: The pouched product of any one of embodiments 4-7, wherein the acid component comprises a

combination of a tricarboxylic acid and a dicarboxylic acid in a weight ratio of from about 2:1 to about 1:2.

Embodiment 9: The pouched product of any one of embodiments 4-8, wherein the acid component is citric acid, tartaric acid, or a combination thereof.

Embodiment 10: The pouched product of any one of embodiments 4-9, wherein the acid component is a combination of citric acid and tartaric acid in a ratio of from about 2:1 to about 1:2 by weight.

Embodiment 11: The pouched product of any one of embodiments 4-10, wherein the base component is a bicarbonate material.

Embodiment 12: The pouched product of any one of embodiments 4-11, wherein the acid component is present in an amount of from about 10% to about 20% by weight, based on the total dry weight of the effervescent material.

Embodiment 13: The pouched product of any one of embodiments 4-12, wherein the acid component and the base component are present in about a 1:1 molar ratio.

Embodiment 14: The pouched product of any one of embodiments 1-13, wherein the at least one active ingredient comprises one or more flavoring agents, botanical materials, stimulants, nicotine components, amino acids, vitamins, cannabinoids, cannabimimetics, terpenes, nutraceuticals, pharmaceutical agents, or combinations thereof.

Embodiment 15: The pouched product of any one of embodiments 1-13, wherein the at least one active ingredient comprises a nicotine component.

Embodiment 16: The pouched product of embodiment 15, wherein the nicotine component is selected from the group consisting of free base nicotine, a nicotine salt, an ion-paired nicotine complex, resin bound nicotine, and combinations thereof.

Embodiment 17: The pouched product of any one of embodiments 1-16, wherein the effervescent material and the filler component are present in the pouch as a mixture of discrete particles of the effervescent material and the filler component.

Embodiment 18: The pouched product of any one of embodiments 1-17, wherein the filler component comprises a cellulosic material.

Embodiment 19: The pouched product of embodiment 18, wherein the cellulosic material is cellulose or microcrystalline cellulose.

Embodiment 20: The pouched product of any one of embodiments 1-19, wherein the at least one active ingredient is adsorbed in or on the filler component.

Embodiment 21: The pouched product of any one of embodiments 18-20, wherein the cellulosic material is in the form of spheres.

Embodiment 22: The pouched product of any one of embodiments 1-21, wherein the effervescent material further comprises a coating, such as a coating comprising a lipid, a cellulose ether, shellac, gum arabic, or a combination thereof.

Embodiment 23: The pouched product of embodiment 22, wherein the lipid is an oil selected from the group consisting of palm oil, palm kernel oil, soybean oil, cottonseed oil, and combinations thereof, wherein the oil may be hydrogenated, partially hydrogenated, or non-hydrogenated.

Embodiment 24: The pouched product of any one of embodiments 1-23, further comprising one or more additives selected from the group consisting of sweeteners, taste modifiers, salts, binders, plasticizers, buffering agents, colorants, humectants, oral care additives, preservatives, disintegration aids, antioxidants, flow aids, and combinations thereof.

Embodiment 25: The pouched product of any one of embodiments 1-24, wherein: the effervescent material is present in granulated form, the effervescent material comprising isomalt, glycerin, an acid component, and a base component; and/or the filler component is present as cellulose spheres, the cellulose spheres comprising cellulose, a nicotine component, one or more salts, one or more sweeteners, and a flavoring agent.

Embodiment 26: The pouched product of embodiment 25, wherein the isomalt is present in an amount in a range from about 38 to about 56% by weight, based on the total weight of the effervescent material, and the glycerin is present in an amount in a range 6 to 10 by weight, based on the total weight of the effervescent material.

Embodiment 27: The pouched product of any one of embodiments 1-26, comprising: from about 40 to about 60 percent by weight of the effervescent material; and from about 40 to about 60 percent by weight of the filler component, based on the total weight of the effervescent composition.

These and other features, aspects, and advantages of the disclosure will be apparent from a reading of the following detailed description together with the accompanying drawing, which are briefly described below. The disclosure includes any combination of two, three, four, or more of the above-noted embodiments as well as combinations of any two, three, four, or more features or elements set forth in this disclosure, regardless of whether such features or elements are expressly combined in a specific embodiment description herein. This disclosure is intended to be read holistically such that any separable features or elements of the disclosure, in any of its various aspects and embodiments, should be viewed as intended to be combinable unless the context clearly dictates otherwise.

## BRIEF DESCRIPTION OF THE DRAWING

Having thus described aspects of the disclosure in the foregoing general terms, reference will now be made to the accompanying drawing, which is not necessarily drawn to scale. The drawing is an example only and should not be construed as limiting the disclosure. The figure is a cross-sectional view of a pouched product embodiment according to an example embodiment of the present disclosure including a pouch or fleece at least partially filled with an effervescent composition according to a non-limiting embodiment of the disclosure.

## DETAILED DESCRIPTION

The present disclosure will now be described more fully hereinafter with reference to example embodiments thereof. These example embodiments are described so that this disclosure will be thorough and complete, and will fully convey the scope of the disclosure to those skilled in the art. Indeed, the disclosure may be embodied in many different forms and should not be construed as limited to the embodiments set forth herein; rather, these embodiments are provided so that this disclosure will satisfy applicable legal requirements.

As used in this specification and the claims, the singular forms "a," "an," and "the" include plural referents unless the context clearly dictates otherwise.

The term "about" used throughout this specification is used to describe and account for small fluctuations. For example, the term "about" can refer to less than or equal to ±10%, such as less than or equal to ±5%, less than or equal to ±2%, less than or equal to ±1%, less than or equal to ±0.5%, less than or equal to ±0.2%, less than or equal to ±0.1% or less than or equal to ±0.05%. All numeric values herein are modified by the term "about," whether or not explicitly indicated. A value modified by the term "about" of course includes the specific value. For instance, "about 5.0" must include 5.0.

Reference to "dry weight percent" or "dry weight basis" refers to weight on the basis of dry ingredients (i.e., all ingredients except water). Reference to "wet weight" refers to the weight of the effervescent composition including water. Unless otherwise indicated, reference to "weight percent" of a composition reflects the total wet weight of the composition (i.e., including water).

In some embodiments, any one or more components of the effervescent composition, and the overall effervescent composition disclosed herein, can be described as a particulate material. As used herein, the term "particulate" refers to a material in the form of a plurality of individual particles. In some embodiments, the particles of a particulate material can be described as substantially spherical or granular. In some embodiments, at least some of the particles of the particulate material can be in the form of an agglomerate of multiple particles.

The particle size of a particulate material may be measured by sieve analysis. As the skilled person will readily appreciate, sieve analysis (otherwise known as a gradation test) is a method used to measure the particle size distribution of a particulate material. Typically, sieve analysis involves a nested column of sieves which comprise screens, such as in the form of wire mesh cloths. A pre-weighed sample may be introduced into the top or uppermost sieve in the column, which has the largest screen openings or mesh size (i.e., the largest pore diameter of the sieve). Each lower sieve in the column has progressively smaller screen openings or mesh sizes than the sieve above. Typically, at the base of the column of sieves is a receiver portion to collect any particles having a particle size smaller than the screen opening size or mesh size of the bottom or lowermost sieve in the column (which has the smallest screen opening or mesh size).

In some embodiments, the column of sieves may be placed on or in a mechanical agitator. The agitator causes the vibration of each of the sieves in the column. The mechanical agitator may be activated for a pre-determined period of time in order to ensure that all particles are collected in the correct sieve. In some embodiments, the column of sieves is agitated for a period of time from 0.5 minutes to 10 minutes, such as from 1 minute to 10 minutes, such as from 1 minute to 5 minutes, such as for approximately 3 minutes. Once the agitation of the sieves in the column is complete, the material collected on each sieve is weighed. The weight of each sample on each sieve may then be divided by the total weight in order to obtain a percentage of the mass retained on each sieve. As the skilled person will readily appreciate, the screen opening sizes or mesh sizes for each sieve in the column used for sieve analysis may be selected based on the granularity or known maximum/minimum particle sizes of the sample to be analyzed. In some embodiments, a column of sieves may be used for sieve analysis, wherein the column comprises from 2 to 20 sieves, such as from 5 to 15 sieves. In some embodiments, a column of sieves may be used for sieve analysis, wherein the column comprises 10 sieves. In some embodiments, the largest screen opening or mesh sizes of the sieves used for sieve analysis may be 1000 μm, such as 500 μm, such as 400 μm, or such as 300 μm.

In some embodiments, any material referenced herein (*e.g.,* filler, effervescent material, active ingredient, and the overall effervescent composition) has particles with a particle size as measured by sieve analysis of no greater than about 1000 μm, such as no greater than about 500 μm, such as no greater than about 400 μm, such as no greater than about 350 μm, such as no greater than about 300 μm, such as no greater than about 250 μm, such as no greater than about 200 μm, such as no greater than about 150 μm, such as no greater than about 100 μm, such as no greater than about 50

μm, such as no greater than about 40 μm, such as no greater than about 30 μm. In some embodiments, at least 60% by weight of the particles of any particulate material referenced herein have a particle size as measured by sieve analysis of no greater than about 1000 μm, such as no greater than about 500 μm, such as no greater than about 400 μm, such as no greater than about 350 μm, such as no greater than about 300 μm, such as no greater than about 250 μm, such as no greater than about 200 μm, such as no greater than about 150 μm, such as no greater than about 100 μm, such as no greater than about 50 μm, such as no greater than about 40 μm, such as no greater than about 30 μm. In some embodiments, at least 70% by weight of the particles of any particulate material referenced herein have a particle size as measured by sieve analysis of no greater than about 1000 μm, such as no greater than about 500 μm, such as no greater than about 400 μm, such as no greater than about 350 μm, such as no greater than about 300 μm, such as no greater than about 250 μm, such as no greater than about 200 μm, such as no greater than about 150 μm, such as no greater than about 100 μm, such as no greater than about 50 μm, such as no greater than about 40 μm, such as no greater than about 30 μm. In some embodiments, at least 80% by weight of the particles of any particulate material referenced herein have a particle size as measured by sieve analysis of no greater than about 1000 μm, such as no greater than about 500 μm, such as no greater than about 400 μm, such as no greater than about 350 μm, such as no greater than about 300 μm, such as no greater than about 250 μm, such as no greater than about 200 μm, such as no greater than about 150 μm, such as no greater than about 100 μm, such as no greater than about 50 μm, such as no greater than about 40 μm, such as no greater than about 30 μm. In some embodiments, at least 90% by weight of the particles of any particulate material referenced herein have a particle size as measured by sieve analysis of no greater than about 1000 μm, such as no greater than about 500 μm, such as no greater than about 400 μm, such as no greater than about 350 μm, such as no greater than about 300 μm, such as no greater than about 250 μm, such as no greater than about 200 μm, such as no greater than about 150 μm, such as no greater than about 100 μm, such as no greater than about 50 μm, such as no greater than about 40 μm, such as no greater than about 30 μm. In some embodiments, at least 95% by weight of the particles of any particulate material referenced herein have a particle size as measured by sieve analysis of no greater than about 1000 μm, such as no greater than about 500 μm, such as no greater than about 400 μm, such as no greater than about 350 μm, such as no greater than about 300 μm, such as no greater than about 250 μm, such as no greater than about 200 μm, such as no greater than about 150 μm, such as no greater than about 100 μm, such as no greater than about 50 μm, such as no greater than about 40 μm, such as no greater than about 30 μm. In some embodiments, at least 99% by weight of the particles of any particulate material referenced herein have a particle size as measured by sieve analysis of no greater than about 1000 μm, such as no greater than about 500 μm, such as no greater than about 400 μm, such as no greater than about 350 μm, such as no greater than about 300 μm, such as no greater than about 250 μm, such as no greater than about 200 μm, such as no greater than about 150 μm, such as no greater than about 100 μm, such as no greater than about 50 μm, such as no greater than about 40 μm, such as no greater than about 30 μm. In some embodiments, approximately 100% by weight of the particles of any particulate material referenced herein have a particle size as measured by sieve analysis of no greater than about 1000 μm, such as no greater than about 500 μm, such as no greater than about 400 μm, such as no greater than about 350 μm, such as no greater than about 300 μm, such as no greater than about 250 μm, such as no greater than about 200 μm, such as no greater than about 150 μm, such as no greater than about 100 μm, such as no greater than about 50 μm, such as no greater than about 40 μm, such as no greater than about 30 μm.

In some embodiments, at least 50% by weight, such as at least 60% by weight, such as at least 70% by weight, such as at least 80% by weight, such as at least 90% by weight, such as at least 95% by weight, such as at least 99% by weight of the particles of any particulate material referenced herein have a particle size as measured by sieve analysis of from about 0.01 μm to about 1000 μm, such as from about 0.05 μm to about 750 μm, such as from about 0.1 μm to about 500 μm, such as from about 0.25 μm to about 500 μm. In some embodiments, at least 50% by weight, such as at least 60% by weight, such as at least 70% by weight, such as at least 80% by weight, such as at least 90% by weight, such as at least 95% by weight, such as at least 99% by weight of the particles of any particulate material referenced herein have a particle size as measured by sieve analysis of from about 10 μm to about 400 μm, such as from about 50 μm to about 350 μm, such as from about 100 μm to about 350 μm, such as from about 200 μm to about 300 μm.

In some embodiments, the effervescent material has a particle size as measured by sieve analysis of less than about 180 μm, such as less than about 150, less than about 120, less than about 80, less than about 60 μm, or less than about 40 μm. In some embodiments, the effervescent material has a particle size as measured by sieve analysis from about 30 to about 180 μm, such as from about 30 to about 40 μm, or from about 50 to about 60 μm. Without wishing to be bound by theory, it is believed that effervescent material particle sizes greater than about 180 μm contribute an unpleasantly rough texture to the effervescent composition. Conversely, effervescent material particle sizes smaller than about 180 μm produce, in some embodiments, a faster onset of effervescence, and provide a smoother texture.

In some embodiments, the particle size as measured by sieve analysis of the remaining effervescent composition components which may be present (e.g., filler, active ingredients, flavorants, sweeteners, taste modifiers, salts, binders, buffering agents, colorants, humectants, oral care additives, preservatives, disintegration aids, antioxidants, flow aids, compressibility aids) is less than about 50 μm, such as less than about 40 μm, less than about 35 μm, or less than about 30 μm. In some embodiments, the particle size as measured by sieve analysis of the remaining effervescent composition

components which may be present is from about 25, about 30, or about 35, to about 40, about 45, or about 50 μm. In some embodiments, the particle size as measured by sieve analysis of the remaining effervescent composition components which may be present is from about 25 to about 35 μm. In some embodiments, the particle size as measured by sieve analysis of the remaining effervescent composition components which may be present is less than about 32 μm.

The pouched product as described herein comprises an effervescent material enclosed in a pouch. The effervescent composition as described herein comprises an effervescent material, a filler component, and at least one active ingredient. The relative amounts of the various components within the effervescent composition may vary, and typically are selected so as to provide the desired sensory and performance characteristics to the product. The example individual components of the effervescent composition and the pouched product are described herein below.

## Effervescent material

The effervescent composition as described herein comprises an effervescent material. As used herein, the term "effervescent material" refers to a material which, upon contact with moisture (e.g., saliva in the oral cavity of a consumer of the effervescent composition as disclosed herein), releases bubbles of a gas resulting in a foaming or fizzing action. The effervescent material can be of two general types: gasified materials which release entrapped gas upon use, and those including chemically reactive components which liberate gas during use. Each type is contemplated herein for use in the disclosed compositions, and each is described further herein below.

### *Gasified material*

In some embodiments, the effervescence may be produced by release of entrapped gas. Accordingly, in some embodiments, the effervescent material comprises a sugar material containing an entrapped gaseous component, such that release of the entrapped gaseous component occurs upon dissolution of the sugar material in the oral cavity. In some embodiments, the sugar material containing an entrapped gaseous component is in the form of a gasified sugar material in particulate form, the gasified sugar material particles being in admixture with the filler and active ingredient. As used herein, "gasified sugar material" refers to a sugar material containing an entrapped gaseous component capable of release upon dissolution of the sugar material in the oral cavity. The gasified sugar material is typically provided in solid form (e.g., granular or particulate form). The average particle size of the gasified sugar material can vary, but is typically about 50 to about 800 microns, more often about 100 to about 600 microns, and most often about 125 to about 500 microns. The gasified sugar material is advantageously maintained in a very dry state to avoid premature effervescence during handling or storage. For example, the gasified sugar material will typically comprise less than about 5% water by weight, less than about 3% water by weight, less than about 2% water by weight, or less than about 1% water by weight.

Commercially available examples of gasified sugar material are sold under the brand name Carbonated Crystals™ by Raven Manufacturing, LLC of Neenah, Wis. Example methods for forming gasified sugar materials are set forth in U.S. Pat. No. 4,289,794 to Kleiner et al.; U.S. Pat. No. 5,165,951 to Gallart et al., and U.S. Pat. No. 5,439,698 to Ahn et al, all of which are incorporated by reference herein. Typical manufacturing processes involve introducing a gaseous component (e.g., carbon dioxide) under pressure (e.g., 50 to 650 psig) to the sugar material while the sugar is in melted form.

The amount of gasified sugar material in the effervescent composition can vary and will depend in part on the desired organoleptic properties of the effervescent composition. Typically, the amount of gasified sugar material (including the total weight of sugar materials and entrapped gas) is in the range of about 10 to about 90 dry weight percent, based on the total weight of the effervescent composition, such as about 20 to about 60 dry weight percent, or about 30 to about 50 dry weight percent.

The sugar component of the gasified sugar material can be any of a variety of monosaccharides (e.g., glucose, fructose, galactose), disaccharides (e.g., sucrose, lactose, maltose), trisaccharides, or oligosaccharides. Although sucrose or other nutritive sweeteners can be used as the sugar material, the effervescent composition of the disclosure can also be prepared as a sugar-free product, meaning the gasified sugar material can be characterized as a sugar substitute. "Sugar-free" as used herein is intended to include products having less than about 1/15th sugar by weight, or less than about 1/10th sugar by weight. In some embodiments, the gasified sugar materials is carbonated isomalt.

### *Effervescent combination*

In some embodiments, the effervescent material comprises a combination of two or more components capable of reacting, typically in an aqueous environment, to produce a gas. The resulting gas is typically carbon dioxide, although it is possible to use reactive couples that produce other gases that are safe for human consumption, such as oxygen. The presence of the effervescent materials adds distinctive organoleptic properties to the product, particularly in terms of taste and mouthfeel. In particular, the presence of effervescent materials masks or alters the perception of bitterness, for

example, of an active ingredient present in the composition.

The use of effervescent materials is described, for example, in U.S. Pat. No. 4,639,368 to Niazi et al.; U.S. Pat. No. 5,178,878 to Wehling et al.; U.S. Pat. No. 5,223,264 to Wehling et al.; U.S. Pat. No. 6,974,590 to Pather et al.; and U.S. Pat. No. 7,381,667 to Bergquist et al., as well as US Pat. Pub. Nos. 2006/0191548 to Strickland et al.; 2009/0025741 to Crawford et al; 2010/0018539 to Brinkley et al.; and 2010/0170522 to Sun et al.; and PCT WO 97/06786 to Johnson et al., all of which are incorporated by reference herein.

In some embodiments, the effervescent material can include an acid/base pair that provides the effervescent effect of the composition. See, for example, the use of acids and bases in effervescent compositions described in U.S. Pat. Pub. No. 2012/0055494 to Hunt et al., which is incorporated by reference herein.

In one embodiment, the effervescent material comprises a reactive couple comprising at least one acid component (an acid, an anhydride, or an acid salt) and at least one base capable of reacting with the acid component to release carbon dioxide. Multiple acids and multiple bases can be combined in the same product to produce the desired reaction.

*Acid component*

In some embodiments, the acid component of the effervescent material is selected from carboxylic acids having about 2 to about 12 carbon atoms (e.g., C2-C10 or C2-C8 or C2-C6 carboxylic acids), wherein the carboxylic acids are monoprotic or polyprotic (e.g., dicarboxylic acids or tricarboxylic acids). Example organic acids include citric acid, malic acid, tartaric acid, succinic acid, adipic acid, fumaric acid, gluconic acid, and combinations thereof. Example acid salts include acidic sodium salts, acidic calcium salts, dihydrogen phosphate salts, and disodium dihydrogen pyrophosphate salts. In some embodiments, the acid component is citric acid or tartaric acid.

In some embodiments, a combination of acids is utilized where at least one acid is a polyprotic acid, such as a dicarboxylic acid (tartaric acid) or a tricarboxylic acid (e.g., citric acid). Combinations of a dicarboxylic acid and a tricarboxylic acid are also suitable for use in the effervescent material. In some embodiments, the acid component comprises a combination of a tricarboxylic acid and a dicarboxylic acid in a weight ratio of from about 2:1 to about 1:2, for example, from about 2:1, about 1.5:1, or about 1:1, to about 1:1.5, or about 1:2. In some embodiments, the acid component is a combination of citric acid and tartaric acid in a ratio of from about 2:1 to about 1:2 by weight. In some embodiments, the acid component is a combination of citric acid and tartaric acid in a 1:1 ratio by weight.

The amount of acid component of the effervescent material in the composition can vary but is typically from about 1 to about 25 dry weight percent, such as about 5 to about 20 dry weight percent, or about 10 to about 18 dry weight percent (e.g., about 10, about 11, about 12, about 13, about 14, about 15, about 16, about 17, or about 18 dry weight percent). Where three or more acids are utilized, each acid is typically present in an amount of about 10 to about 35 dry weight percent, based on the total weight of the acid component. In some embodiment, the acid component is 5% by weight of citric acid and 5% by weight tartaric acid. The acid component, e.g., citric and/or tartaric acid particles, may be encapsulated or coated.

*Base component*

Examples of suitable base components include carbonate and bicarbonate materials, particularly alkali metal or alkaline earth metal salts thereof. Carbonate and bicarbonate base materials capable of use in the present disclosure include sodium carbonate, sodium bicarbonate, potassium carbonate, potassium bicarbonate, magnesium carbonate, calcium carbonate, sodium sesquicarbonate, sodium glycine carbonate, lysine carbonate, and arginine carbonate. In some embodiments, the base component is sodium bicarbonate. The base component, e.g., sodium bicarbonate particles may be encapsulated or coated. Encapsulated sodium bicarbonate is available from, for example Watson (301 Heffernan Drive West Haven, CT 06516) or Clabber Girl Corporation (900 Wabash Ave, Terre Haute, IN 47807).

In some embodiments, a combination of carbonate salts and bicarbonate components may be used. Bicarbonate materials, while highly reactive in effervescent reactions, are not efficient buffering agents in certain desirable pH ranges. Thus, in some embodiments utilizing both a bicarbonate and carbonate material, it is advantageous to stoichiometrically match the bicarbonate amount to the acid component of the effervescent material and use a carbonate material as the main buffering agent. In this manner, although the carbonate material would be expected to participate in the effervescent reaction to a limited degree, the bicarbonate material is present in an amount sufficient to fully react with the available acid component and the carbonate material is present in an amount sufficient to provide the desired pH range.

The amount of the base component (e.g., carbonate or bicarbonate materials) of the effervescent material in the effervescent composition can vary, but is typically about 4 to about 30 dry weight percent, for example, from about 5 to about 25 dry weight percent, about 8 to about 20 dry weight percent, or about 6 to about 12 dry weight percent (e.g., about 4, about 6, about 8, about 10, about 12, about 14, about 16, about 18, or about 20 dry weight percent). In some embodiments, the effervescent composition may include both a carbonate component and a bicarbonate component. For some

embodiments, the amount of carbonate material can vary, but is typically about 3 to about 20 dry weight percent, such as about 5 to about 15 dry weight percent, or about 8 to about 15 dry weight percent (e.g., about 8, about 9, about 10, about 11, about 12, about 13, or about 14 dry weight percent). For some embodiments, the amount of bicarbonate material can vary, but is typically about 3 to about 20 dry weight percent, often about 5 to about 15 dry weight percent, and most often about 8 to about 15 dry weight percent (e.g., about 8, about 9, about 10, about 11, about 12, about 13, or about 14 dry weight percent). In some embodiments, the base component is sodium bicarbonate, present in an amount by weight of about 12%, based on the total weight of the effervescent composition.

In some embodiments, it is desirable for the reaction between the acid and base component to proceed completely. To ensure this result, the relevant amount of acid and base can be adjusted so that the necessary equivalent amounts are present. In some embodiments, the acid component and the base component are present in about a 1:1 molar ratio. For example, if a dicarboxylic acid is used, then either a di-reactive base can be used in a roughly equivalent amount, or a monoreactive base could be used at a level roughly twice that of the acid. Likewise, if a tricarboxylic acid is used, then either a tri-reactive base can be used in a roughly equivalent amount, or a monoreactive base could be used at a level roughly thrice that of the acid. Alternatively, an excess amount of either acid or base can be used, particularly where the acid or base is intended to provide an independent effect on the organoleptic properties of the effervescent composition beyond simply providing effervescence.

The amount of total effervescent material (i.e., all reactive materials that produce the gaseous product) in the effervescent composition can vary. The amount of such material should be sufficient to enable the effervescent composition to effervesce when placed in the oral cavity. The amount of effervescent material is typically about 5 to about 50 dry weight percent, for example, from about 8 to about 30 dry weight percent, about 10 to about 25 dry weight percent (e.g., about 10, about 12, about 14, about 16, about 18, about 20, or about 22 dry weight percent), based on the total weight of the effervescent composition. The amount of effervescent material in some embodiments can be characterized as at least about 10 dry weight percent, or at least about 15 dry weight percent, or at least about 20 dry weight percent, or at least about 25 dry weight percent. The amount of effervescent material in some embodiments can be characterized as no more than about 50 dry weight percent, no more than about 40 dry weight percent, no more than about 35 dry weight percent, no more than about 30 dry weight percent, or no more than about 20 dry weight percent.

The amount of gas (e.g., carbon dioxide) that evolves from the effervescence reaction in the effervescent composition can vary and depends in part on the desired sensory characteristics of the effervescent composition. The amount of effervescent material can be selected to achieve the desired level of carbon dioxide release. One method for measuring the amount of carbon dioxide released from a given quantity of effervescent composition involves the following steps: (1) pipetting 1 ml of water to a vial; (2) capping the vial; (3) pre-weighing the capped vial using, for example, a Mettler Model AE163 balance or equivalent analytical balance readable to 0.0001 g; (4) reweighing the capped vial along with the effervescent composition to be tested; (5) add the effervescent composition to the water in the vial and cap the vial loosely (tighten cap until barely tight and then loosen cap slightly); (6) after about thirty minutes, vortexing the vials for 3-4 seconds using a vortex mixer such as a Fisher Scientific Touch Mixer Model 232 or equivalent; (8) loosening cap to release trapped gas and then again capping vial loosely; (9) after about one hour, repeating Steps 7 and 8 and reweighing vial; and (10) after about 1.5 hours, repeat Steps 7 and 8 and reweighing vial. The amount of carbon dioxide evolved from the effervescent composition is the difference in weight between Step 4 to Step 10.

In the above test, the intent is to use enough water in the vial to initiate the reaction between acid and base, but not so much that an appreciable amount of carbon dioxide remains dissolved in the water. Vortexing the sample agitates the liquid to overcome supersaturation of the water with carbon dioxide. The vials are loosely capped to allow carbon dioxide to escape without allowing water to evaporate. Carbon dioxide is heavier than air, so weights at different time points are taken to make sure that the carbon dioxide has diffused out of the head space of the vial. The last two vial weights should agree within about 1.5 mg.

The amount of evolved carbon dioxide from an effervescent composition of the disclosure can be expressed as a ratio of weight of carbon dioxide evolved to total effervescent composition weight. In some embodiments, this ratio can be from about 10 μg carbon dioxide/mg of effervescent composition to about 120 μg carbon dioxide/mg of effervescent composition, from about 10 μg carbon dioxide/mg to about 60 μg carbon dioxide/mg, or from about 10 μg carbon dioxide/mg to about 30 μg carbon dioxide/mg. In some embodiments, the amount of evolved carbon dioxide can be characterized as at least about 10 μg carbon dioxide/mg of effervescent composition, or at least about 15 μg carbon dioxide/mg of effervescent composition.

*Binder*

In some embodiments, the effervescent material comprises a binder. Combinations of binders can also be used. Examples of potential binders include cellulosic materials, maltodextrin, sugars, and sugar alcohols. In some embodiments, the binder includes at least one sugar alcohol. Sugar alcohols are polyols derived from monosaccharides or disaccharides that have a partially or fully hydrogenated form. Sugar alcohols have, for example, about 4 to about 20

carbon atoms and include erythritol, arabitol, ribitol, isomalt, maltitol, dulcitol, iditol, mannitol, xylitol, lactitol, sorbitol, and combinations thereof (e.g., hydrogenated starch hydrolysates). In some embodiments, the binder comprises at least one of mannitol, maltodextrin, isomalt, and starch-derived polysaccharides. In some embodiments, the binder comprises isomalt.

The amount of binder can vary but is typically added in an amount sufficient to hold together the acid and base components. In some embodiments, the binder is present in an amount greater than about 25%, and up to about 75% of the effervescent composition by weight, based on the total weight of the effervescent material. A typical range of filler within the effervescent material can be from about 25 to about 75% by total weight of the effervescent composition, for example, from about 25, about 30, about 35, about 40, about 45, or about 50%, to about 55, about 60%, about 65, about 70%, or about 75% by weight. In some embodiments, the amount of binder is at least about 30% by weight, such as at least about 35%, or at least about 40%, or at least about 45%, or at least about 50%, or at least about 55%, based on the total weight of the effervescent material. In some embodiments, the binder is present in a total amount of up to about 60% by weight, based on the total weight of the effervescent material. In some embodiments, the binder is present in an amount in a range from about 38% to about 56% by weight, based on the total weight of the effervescent material. In some embodiments, the binder includes at least one sugar alcohol. In some embodiments, the binder is isomalt, present in an amount by weight in a range from about 38 to about 56%, such as about 38, about 40, about 42, about 44, about 46, about 48, about 50, about 52, about 54, or about 56% by weight, based on the total weight of the effervescent material.

*Humectant*

In some embodiments, the effervescent material comprises a humectant. Examples of humectants include, but are not limited to, glycerol, propylene glycol, and the like. Where included, the humectant is typically provided in an amount sufficient to impart the desired physical characteristics to the effervescent material. Further, in some instances, the humectant may impart desirable processing characteristics to the effervescent material, such as allowing extrusion and spheronization for forming beads.

When present, a humectant will typically make up about 10% or less of the weight of the effervescent material (e.g., from about 5 to about 10% by weight), for example, from about 5%, about 6, or about 7, to about 8, about 9, or about 10% by weight, based on the total weight of the effervescent material.

In some embodiments, the effervescent material comprises a combination of a binder and a humectant. In some embodiments, the binder is a sugar alcohol. In some embodiments, the humectant is glycerol. In some embodiments, the combination forms an extrudable material which can be shaped. In some embodiments, the binder is isomalt and the humectant is glycerin, which, together with the remaining effervescent material components, form an extrudable dough which can desirably be formed into beads.

**Filler Component**

The effervescent composition as described herein comprises a filler component. Such fillers may fulfill multiple functions, such as enhancing certain organoleptic properties (e.g., texture and mouthfeel), enhancing cohesiveness or compressibility of the composition, and the like. Combinations of fillers can also be used. Examples of potential fillers include cellulosic materials, maltodextrin, dextrose, calcium carbonate, calcium phosphate, lactose, and sugar alcohols. In some embodiments, the filler includes at least one sugar alcohol. Sugar alcohols are polyols derived from monosaccharides or disaccharides that have a partially or fully hydrogenated form. Sugar alcohols have, for example, about 4 to about 20 carbon atoms and include erythritol, arabitol, ribitol, isomalt, maltitol, dulcitol, iditol, mannitol, xylitol, lactitol, sorbitol, and combinations thereof (e.g., hydrogenated starch hydrolysates). In some embodiments, the filler comprises at least one of mannitol, maltodextrin, isomalt, and starch-derived polysaccharides.

In some embodiments, the filler is a porous particulate material and is cellulose-based. For example, suitable fillers are any non-tobacco plant material or derivative thereof, including cellulose materials derived from such sources. Examples of cellulosic non-tobacco plant material include cereal grains (e.g., maize, oat, barley, rye, buckwheat, and the like), sugar beet (e.g., FIBREX® brand filler available from International Fiber Corporation), bran fiber, and mixtures thereof. Non-limiting examples of derivatives of non-tobacco plant material include starches (e.g., from potato, wheat, rice, corn), natural cellulose, and modified cellulosic materials. "Starch" as used herein may refer to pure starch from any source, modified starch, or starch derivatives. Starch is present, typically in granular form, in almost all green plants and in various types of plant tissues and organs (e.g., seeds, leaves, rhizomes, roots, tubers, shoots, fruits, grains, and stems). Starch can vary in composition, as well as in granular shape and size. Often, starch from different sources has different chemical and physical characteristics. A specific starch can be selected for inclusion in the effervescent composition based on the ability of the starch material to impart a specific organoleptic property to the effervescent composition. Starches derived from various sources can be used. For example, major sources of starch include cereal grains (e.g., rice, wheat, and maize) and root vegetables (e.g., potatoes and cassava). Other examples of sources of starch include acorns,

arrowroot, arracacha, bananas, barley, beans (e.g., favas, lentils, mung beans, peas, chickpeas), breadfruit, buckwheat, canna, chestnuts, colacasia, katakuri, kudzu, malanga, millet, oats, oca, Polynesian arrowroot, sago, sorghum, sweet potato, quinoa, rye, tapioca, taro, tobacco, water chestnuts, and yams. Certain starches are modified starches. A modified starch has undergone one or more structural modifications, often designed to alter its high heat properties. Some starches have been developed by genetic modifications and are considered to be "modified" starches. Other starches are obtained and subsequently modified. For example, modified starches can be starches that have been subjected to chemical reactions, such as esterification, etherification, oxidation, depolymerization (thinning) by acid catalysis or oxidation in the presence of base, bleaching, transglycosylation and depolymerization (e.g., dextrinization in the presence of a catalyst), cross-linking, enzyme treatment, acetylation, hydroxypropylation, and/or partial hydrolysis. Other starches are modified by heat treatments, such as pregelatinization, dextrinization, and/or cold-water swelling processes. Certain modified starches include monostarch phosphate, distarch glycerol, distarch phosphate esterified with sodium trimetaphosphate, phosphate distarch phosphate, acetylated distarch phosphate, starch acetate esterified with acetic anhydride, starch acetate esterified with vinyl acetate, acetylated distarch adipate, acetylated distarch glycerol, hydroxypropyl starch, hydroxypropyl distarch glycerol, and starch sodium octenyl succinate.

In some embodiments, the filler comprises or is an inorganic material. Examples of potential inorganic fillers include calcium carbonate, calcium phosphate, and bioceramic materials (e.g., porous hydroxyapatite).

In some embodiments, the filler comprises a cellulose material or a cellulose derivative, such as microcrystalline cellulose ("mcc"). The mcc may be synthetic or semi-synthetic, or it may be obtained entirely from natural celluloses. The mcc may be selected from the group consisting of AVICEL® grades PH-100, PH-102, PH-103, PH-105, PH-112, PH-113, PH-200, PH-300, PH-302, VIVACEL® grades 101, 102, 12, 20 and EMOCEL® grades 50M and 90M, and the like, and mixtures thereof.

In some embodiments of the present disclosure, a substantially spherical filler in particulate form is utilized, and such fillers can be defined by their sphericity, which is a measure of how closely an object resembles a perfect sphere. Sphericity ($\Psi$) can be measuring using the equation below, wherein $V_p$ is the volume of the object and $A_p$ is the surface area of the object.

$$\Psi = (\pi^{1/3}(6V_p)^{2/3})/A_p$$

The sphericity of a sphere is unity by definition and any shape that is not a perfect sphere will have a sphericity less than 1. In some embodiments, the sphericity of the substantially spherical fillers of the present disclosure will be about 0.7 or higher, such as about 0.8 or higher or about 0.9 or higher (e.g., about 0.7 to 1 or about 0.75 to 1 or about 0.8 to 1 or about 0.85 to 1, or about 0.9 to 1).

In some embodiments, the substantially spherical filler comprises microcrystalline cellulose ("MCC"). The MCC may be synthetic or semi-synthetic, or it may be obtained entirely from natural celluloses. By "substantially spherical MCC" is meant a material comprising, consisting essentially of, or consisting of MCC, wherein the material is a substantially spherical particulate filler component as referenced herein above.

The average diameter (mean) or D50 (median) particle size of the substantially spherical particulate filler particles provided herein can vary, and is not particularly limited. For example, in some embodiments, the spherical filler particles have an average diameter and/or a D50 value of about 100 μm to about 2000 μm, such as about 250 μm to about 750 μm. For example, in some embodiments, the average diameter is about 100 μm to about 500 μm, e.g., about 100 μm to about 400 μm, about 100 μm to about 300 μm, about 100 μm to about 200 μm, about 200 μm to about 500 μm, about 200 μm to about 400 μm, about 200 μm to about 300 μm, about 300 μm to about 500 μm, about 300 μm to about 400 μm, or about 400 μm to about 500 μm. In some embodiments, the average diameter is about 500 μm to about 1000 μm, e.g., about 500 μm to about 900 μm, about 500 μm to about 800 μm, about 500 μm to about 700 μm, about 500 μm to about 600 μm, about 600 μm to about 1000 μm, about 600 μm to about 900 μm, about 600 μm to about 800 μm, about 600 μm to about 700 μm, about 700 μm to about 1000 μm, about 700 μm to about 900 μm, about 700 μm to about 800 μm, about 800 μm to about 1000 μm, about 800 μm to about 900 μm, or about 900 μm to about 1000 μm. In some embodiments, the substantially spherical filler component has an average diameter and/or D50 value of about 300 to 650 microns, such as about 350 to about 500 microns.

The distribution of diameters around this average diameter (i.e., the particle size distribution) can also vary; in some embodiments, the distribution of diameters is close to the listed value (e.g., +/- about 25% of the stated value, +/- about 20% of the stated value, +/- about 15% of the stated value, +/- about 10% of the stated value, +/- about 5% of the stated value, or +/- about 1% of the stated value. The disclosure is not, however, limited to materials with such narrow distributions; in some embodiments, the diameter of the MCC spheres within a given material can vary within a wider range. Particle size distributions can be determined using a sieve analysis.

In some embodiments, the substantially spherical filler component comprises MCC. In some embodiments, the substantially spherical filler component comprises solid (although porous) MCC spheres. In some embodiments, the

substantially spherical filler component comprises hollow MCC spheres. In some embodiments, the center/core of such hollow MCC spheres may be unfilled; in some embodiments, the center/core of such hollow MCC spheres may be filled with one or more additional components (e.g., flavorants, fillers, active ingredients, etc.). Examples of suitable MCC spheres include, but are not limited to, Vivapur® MCC spheres from JRS Pharma, available, e.g., with particle sizes of 100-200 μm (Vivapur® 100), 200-355 μm (Vivapur® 200), 355-500 μm (Vivapur® 350), 500-710 μm (Vivapur® 500), 710-1000 μm (Vivapur®700), and 1000-1400 μm (Vivapur® 1000). Further examples of suitable MCC spheres include, but are not limited to, Celphere™ MCC spheres from Asahi Kasei Corporation, available, e.g., with particle sizes of 75-212 μm (Celphere™ SCP-100), 106-212 μm (Celphere™ CP-102), 150-300 μm (Celphere™ CP-203), 300-500 μm (Celphere™ CP-305), and 500-710 μm (Celphere™ CP-507).

The amount of filler component can vary, but is typically greater than about 25%, and up to about 75% of the effervescent composition by weight, based on the total weight of the composition. A typical range of filler within the effervescent composition can be from about 25 to about 75% by total weight of the effervescent composition, for example, from about 25, about 30, about 35, about 40, about 45, or about 50%, to about 55, about 60%, about 65, about 70%, or about 75% by weight (e.g., about 20 to about 60%, or about 25 to about 55%, or from about 30 to about 50% by weight). In some embodiments, the amount of filler is in a range from about 32 to about 48% by weight, such as from about 32, about 34, about 36, about 38, or about 40, to about 42, about 44, about 46, or about 48% by weight, based on the total weight of the effervescent composition.

## Active ingredient

The effervescent composition as disclosed herein comprises at least one active ingredient. As used herein, an "active ingredient" refers to one or more substances belonging to any of the following categories: API (active pharmaceutical substances), food additives, natural medicaments, and naturally occurring substances that can have an effect on humans. Example active ingredients include any ingredient known to impact one or more biological functions within the body, such as ingredients that furnish pharmacological activity or other direct effect in the diagnosis, cure, mitigation, treatment, or prevention of disease, or which affect the structure or any function of the body of humans (e.g., provide a stimulating action on the central nervous system, have an energizing effect, an antipyretic or analgesic action, or an otherwise useful effect on the body). In some embodiments, the active ingredient may be of the type generally referred to as dietary supplements, nutraceuticals, "phytochemicals" or "functional foods". These types of additives are sometimes defined in the art as encompassing substances typically available from naturally occurring sources (e.g., botanical materials) that provide one or more advantageous biological effects (e.g., health promotion, disease prevention, or other medicinal properties), but are not classified or regulated as drugs.

Non-limiting examples of active ingredients include those falling in the categories of botanical ingredients, stimulants, amino acids, nicotine components, and/or pharmaceutical, nutraceutical, and medicinal ingredients (e.g., vitamins, such as A, B3, B6, B12, and C, and/or cannabinoids, such as tetrahydrocannabinol (THC) and cannabidiol (CBD)). Each of these categories is further described herein below. The particular choice of active ingredients will vary depending upon the desired flavor, texture, and desired characteristics of the particular product.

The particular percentages of active ingredients present will vary depending upon the desired characteristics of the particular product. Typically, an active ingredient or combination thereof is present in a total concentration of at least about 0.001% by weight of the effervescent composition, such as in a range from about 0.001% to about 20%. In some embodiments, the active ingredient or combination of active ingredients is present in a concentration from about 0.1% w/w to about 10% by weight, such as, e.g., from about from about 0.5% w/w to about 10%, from about 1% to about 10%, or from about 1% to about 5% by weight, based on the total weight of the effervescent composition. In some embodiments, the active ingredient or combination of active ingredients is present in a concentration of from about 0.001%, about 0.01%, about 0.1% , or about 1%, up to about 20% by weight, such as, e.g., from about from about 0.001%, about 0.002%, about 0.003%, about 0.004%, about 0.005%, about 0.006%, about 0.007%, about 0.008%, about 0.009%, about 0.01%, about 0.02%, about 0.03%, about 0.04%, about 0.05%, about 0.06%, about 0.07%, about 0.08%, about 0.09%, about 0.1%, about 0.2%, about 0.3%, about 0.4%, about 0.5% about 0.6%, about 0.7%, about 0.8%, or about 0.9%, to about 1%, about 2%, about 3%, about 4%, about 5%, about 6%, about 7%, about 8%, about 9%, about 10%, about 11%, about 12%, about 13%, about 14%, about 15%, about 16%, about 17%, about 18%, about 19%, or about 20% by weight, based on the total weight of the effervescent composition. Further suitable ranges for specific active ingredients are provided herein below.

### *Botanical*

In some embodiments, the active ingredient comprises a botanical ingredient. As used herein, the term "botanical ingredient" or "botanical" refers to any plant material or fungal-derived material, including plant material in its natural form and plant material derived from natural plant materials, such as extracts or isolates from plant materials or treated plant materials (e.g., plant materials subjected to heat treatment, fermentation, bleaching, or other treatment processes capable

of altering the physical and/or chemical nature of the material). For the purposes of the present disclosure, a "botanical" includes, but is not limited to, "herbal materials," which refer to seed-producing plants that do not develop persistent woody tissue and are often valued for their medicinal or sensory characteristics (e.g., teas or tisanes). Reference to botanical material as "non-tobacco" is intended to exclude tobacco materials (i.e., does not include any Nicotiana species).

In some embodiments, the compositions as disclosed herein can be characterized as free of any tobacco material (e.g., any embodiment as disclosed herein may be completely or substantially free of any tobacco material). By "substantially free" is meant that no tobacco material has been intentionally added. For example, some embodiments can be characterized as having less than 0.001% by weight of tobacco, or less than 0.0001%, or even 0% by weight of tobacco.

When present, a botanical is typically at a concentration of from about 0.01% w/w to about 10% by weight, such as, e.g., from about from about 0.01% w/w, about 0.05%, about 0.1%, or about 0.5%, to about 1%, about 2%, about 3%, about 4%, about 5%, about 6%, about 7%, about 8%, about 9%, or about 10%, about 11%, about 12%, about 13%, about 14%, or about 15% by weight, based on the total weight of the effervescent composition.

The botanical materials useful in the present disclosure may comprise, without limitation, any of the compounds and sources set forth herein, including mixtures thereof. Certain botanical materials of this type are sometimes referred to as dietary supplements, nutraceuticals, "phytochemicals" or "functional foods." Certain botanicals, as the plant material or an extract thereof, have found use in traditional herbal medicine, and are described further herein.

Non-limiting examples of non-tobacco botanical materials include without limitation acai berry (Euterpe oleracea martius), acerola (Malpighia glabra), alfalfa, allspice, Angelica root, anise (e.g., star anise), annatto seed, apple (Malus domestica), apricot oil, ashwagandha, Bacopa monniera, baobab, basil (Ocimum basilicum), bay, bee balm, beet root, bergamot, blackberry (Morus nigra), black cohosh, black pepper, black tea, blueberries, boldo (Peumus boldus), borage, bugleweed, cacao, calamus root, camu (Myrcaria dubia), cannabis/hemp, caraway seed, cardamom, cassis, catnip, catuaba, cayenne pepper, Centella asiatica, chaga mushroom, Chai-hu, chamomile, cherry, chervil, chive, chlorophyll, chocolate, cilantro, cinnamon (Cinnamomum cassia), citron grass (Cymbopogon citratus), citrus, clary sage, cloves, coconut (Cocos nucifera), coffee, comfrey leaf and root, cordyceps, coriander seed, cranberry, cumin, curcumin, damiana, dandelion, Dorstenia arifolia, Dorstenia odorata, Echinacea, elderberry, elderflower, endro (Anethum graveolens), evening primrose, eucalyptus, fennel, feverfew, flax, Galphimia glauca, garlic, ginger (Zingiber officinale), gingko biloba, ginseng, goji berries, goldenseal, grape seed, grapefruit, grapefruit rosé (Citrus paradisi), graviola (Annona muricata), green tea, guarana, gutu kola, hawthorn, hazel, hemp, hibiscus flower (Hibiscus sabdariffa), honeybush, hops, jiaogulan, jambu (Spilanthes oleraceae), jasmine (Jasminum officinale), juniper berry (Juniperus communis), Kaempferia parviflora (Thai ginseng), kava, laurel, lavender, lemon (Citrus limon), lemon balm, lemongrass, licorice, lilac, Lion's mane, lutein, maca (Lepidium meyenii), mace, marjoram, matcha, milk thistle, mints (menthe), mulberry, Nardostachys chinensis, nutmeg, olive, oolong tea, orange (Citrus sinensis), oregano, papaya, paprika, pennyroyal, peppermint (Mentha piperita), pimento, potato peel, primrose, quercetin, quince, red clover, resveratrol, Rhizoma gastrodiae, Rhodiola, rooibos (red or green), rosehip (Rosa canina), rosemary, saffron, sage, Saint John's Wort, sandalwood, salvia (Salvia officinalis), savory, saw palmetto, Sceletium tortuosum, Schisandra, silybum marianum, Skullcap, spearmint, Spikenard, spirulina, slippery elm bark, sorghum bran hi-tannin, sorghum grain hi-tannin, spearmint (Mentha spicata), spirulina, star anise, sumac bran, tarragon, thyme, tisanes, turmeric, Turnera aphrodisiaca, uva ursi, valerian, vanilla, Viola odorata, wild yam root, wintergreen, withania somnifera, yacon root, yellow dock, yerba mate, and yerba santa.

### *Stimulants*

In some embodiments, the active ingredient comprises one or more stimulants. As used herein, the term "stimulant" refers to a material that increases activity of the central nervous system and/or the body, for example, enhancing focus, cognition, vigor, mood, alertness, and the like. Non-limiting examples of stimulants include caffeine, theacrine, theobromine, and theophylline. Theacrine (1,3,7,9-tetramethyluric acid) is a purine alkaloid which is structurally related to caffeine, and possesses stimulant, analgesic, and anti-inflammatory effects. Present stimulants may be natural, naturally derived, or wholly synthetic. For example, certain botanical materials (guarana, tea, coffee, cocoa, and the like) may possess a stimulant effect by virtue of the presence of e.g., caffeine or related alkaloids, and accordingly are "natural" stimulants. By "naturally derived" is meant the stimulant (e.g., caffeine, theacrine) is in a purified form, outside its natural (e.g., botanical) matrix. For example, caffeine can be obtained by extraction and purification from botanical sources (e.g., tea). By "wholly synthetic", it is meant that the stimulant has been obtained by chemical synthesis. In some embodiments, the active ingredient comprises caffeine. In some embodiments, the active ingredient is caffeine. In some embodiments, the caffeine is present in an encapsulated form. On example of an encapsulated caffeine is Vitashure®, available from Balchem Corp., 52 Sunrise Park Road, New Hampton, NY, 10958.

When present, a stimulant or combination of stimulants (e.g., caffeine, theacrine, and combinations thereof) is typically at a concentration of from about 0.1% w/w to about 15% by weight, such as, e.g., from about from about 0.1% w/w, about 0.2%, about 0.3%, about 0.4%, about 0.5% about 0.6%, about 0.7%, about 0.8%, or about 0.9%, to about 1%, about 2%, about 3%, about 4%, about 5%, about 6%, about 7%, about 8%, about 9%, about 10%, about 11%, about 12%, about

13%, about 14%, or about 15% by weight, based on the total weight of the effervescent composition.

### *Amino acids*

In some embodiments, the active ingredient comprises an amino acid. As used herein, the term "amino acid" refers to an organic compound that contains amine (-NH2) and carboxyl (-COOH) or sulfonic acid (SO3H) functional groups, along with a side chain (R group), which is specific to each amino acid. Amino acids may be proteinogenic or non-proteinogenic. By "proteinogenic" is meant that the amino acid is one of the twenty naturally occurring amino acids found in proteins. The proteinogenic amino acids include alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine, and valine. By "non-proteinogenic" is meant that either the amino acid is not found naturally in protein or is not directly produced by cellular machinery (e.g., is the product of post-translational modification). Non-limiting examples of non-proteinogenic amino acids include gamma-aminobutyric acid (GABA), taurine (2-aminoethanesulfonic acid), theanine (L-γ-glutamylethylamide), hydroxyproline, and beta-alanine.

When present, an amino acid or combination of amino acids (e.g., taurine, theanine, GABA, or combinations thereof) is typically at a concentration of from about 0.1% w/w to about 15% by weight, such as, e.g., from about from about 0.1% w/w, about 0.2%, about 0.3%, about 0.4%, about 0.5% about 0.6%, about 0.7%, about 0.8%, or about 0.9%, to about 1%, about 2%, about 3%, about 4%, about 5%, about 6%, about 7%, about 8%, about 9%, about 10%, about 11%, about 12%, about 13%, about 14%, or about 15% by weight, based on the total weight of the effervescent composition.

### *Vitamins and Minerals*

In some embodiments, the active ingredient comprises a vitamin or combination of vitamins. As used herein, the term "vitamin" refers to an organic molecule (or related set of molecules) that is an essential micronutrient needed for the proper functioning of metabolism in a mammal. There are thirteen vitamins required by human metabolism, which are: vitamin A (as all-trans-retinol, all-trans-retinyl-esters, as well as all-trans-beta-carotene and other provitamin A carotenoids), vitamin B1 (thiamine), vitamin B2 (riboflavin), vitamin B3 (niacin), vitamin B5 (pantothenic acid), vitamin B6 (pyridoxine), vitamin B7 (biotin), vitamin B9 (folic acid or folate), vitamin B12 (cobalamins), vitamin C (ascorbic acid), vitamin D (calciferols), vitamin E (tocopherols and tocotrienols), and vitamin K (quinones). In some embodiments, the active ingredient comprises vitamin C. In some embodiments, the active ingredient is a combination of vitamin C, caffeine, and taurine. In some embodiments, the active ingredient comprises one or more of vitamin B6 and B12. In some embodiments, the active ingredient comprises theanine and one or more of vitamin B6 and B12.

When present, a vitamin or combination of vitamins (e.g., vitamin B6, vitamin B12, vitamin E, vitamin C, or a combination thereof) is typically at a concentration of from about 0.01% w/w to about 1% by weight, such as, e.g., from about 0.01%, about 0.02%, about 0.03%, about 0.04%, about 0.05%, about 0.06%, about 0.07%, about 0.08%, about 0.09%, or about 0.1% w/w, to about 0.2%, about 0.3%, about 0.4%, about 0.5% about 0.6%, about 0.7%, about 0.8%, about 0.9%, or about 1% by weight, based on the total weight of the composition.

In some embodiments, the active ingredient comprises vitamin A. In some embodiments, the vitamin A is encapsulated. In some embodiments, the vitamin is vitamin B6, vitamin B12, vitamin E, vitamin C, or a combination thereof.

In some embodiments, the active ingredient comprises a mineral. As used herein, the term "mineral" refers to an inorganic molecule (or related set of molecules) that is an essential micronutrient needed for the proper functioning of various systems in a mammal. Non-limiting examples of minerals include iron, zinc, copper, selenium, chromium, cobalt, manganese, calcium, phosphorus, sulfur, magnesium, and the like. In some embodiments, the active ingredient comprises iron. Suitable sources of iron include, but are not limited to, ferrous salts such as ferrous sulfate and ferrous gluconate. In some embodiments, the iron is encapsulated.

### *Nicotine component*

In some embodiments, the active ingredient comprises a nicotine component. By "nicotine component" is meant any suitable form of nicotine (e.g., free base or salt) for providing oral absorption of at least a portion of the nicotine present. Typically, the nicotine component is selected from the group consisting of nicotine free base and a nicotine salt. In some embodiments, the nicotine component is nicotine in its free base form, which easily can be adsorbed in for example, a microcrystalline cellulose material to form a microcrystalline cellulose-nicotine carrier complex. See, for example, the discussion of nicotine in free base form in US Pat. Pub. No. 2004/0191322 to Hansson, which is incorporated herein by reference.

In some embodiments, at least a portion of the nicotine component can be employed in the form of a salt. Salts of nicotine can be provided using the types of ingredients and techniques set forth in US Pat. No. 2,033,909 to Cox et al. and

Perfetti, Beitrage Tabakforschung Int., 12: 43-54 (1983), which are incorporated herein by reference. Additionally, salts of nicotine are available from sources such as Pfaltz and Bauer, Inc. and K&K Laboratories, Division of ICN Biochemicals, Inc. Typically, the nicotine component is selected from the group consisting of nicotine free base, a nicotine salt such as hydrochloride, dihydrochloride, monotartrate, bitartrate, sulfate, salicylate, and nicotine zinc chloride.

In some embodiments, at least a portion of the nicotine can be in the form of a resin complex of nicotine, where nicotine is bound in an ion-exchange resin, such as nicotine polacrilex, which is nicotine bound to, for example, a polymethacrilic acid, such as Amberlite IRP64, Purolite C115HMR, or Doshion P551. See, for example, US Pat. No. 3,901,248 to Lichtneckert et al., which is incorporated herein by reference. Another example is a nicotine-polyacrylic carbomer complex, such as with Carbopol 974P. In some embodiments, nicotine may be present in the form of a nicotine polyacrylic complex.

Typically, the nicotine component (calculated as the free base) when present, is in a concentration of at least about 0.001% by weight of the effervescent composition, such as in a range from about 0.001% to about 10%. In some embodiments, the nicotine component is present in a concentration from about 0.1% w/w to about 10% by weight, such as, e.g., from about from about 0.1% w/w, about 0.2%, about 0.3%, about 0.4%, about 0.5% about 0.6%, about 0.7%, about 0.8%, or about 0.9%, to about 1%, about 2%, about 3%, about 4%, about 5%, about 6%, about 7%, about 8%, about 9%, or about 10% by weight, calculated as the free base and based on the total weight of the effervescent composition. In some embodiments, the nicotine component is present in a concentration from about 0.1% w/w to about 3% by weight, such as, e.g., from about from about 0.1% w/w to about 2.5%, from about 0.1% to about 2.0%, from about 0.1% to about 1.5%, or from about 0.1% to about 1% by weight, calculated as the free base and based on the total weight of the composition.

In some embodiments, the products or compositions of the disclosure can be characterized as completely free or substantially free of any nicotine component (e.g., any embodiment as disclosed herein may be completely or substantially free of any nicotine component). By "substantially free" is meant that no nicotine has been intentionally added, beyond trace amounts that may be naturally present in e.g., a botanical material. For example, some embodiments can be characterized as having less than 0.001% by weight of nicotine, or less than 0.0001%, or even 0% by weight of nicotine, calculated as the free base.

In some embodiments, the active ingredient comprises a nicotine component (e.g., any effervescent composition of the disclosure, in addition to comprising any active ingredient or combination of active ingredients as disclosed herein, may further comprise a nicotine component).

### *Cannabinoids*

In some embodiments, the active ingredient comprises one or more cannabinoids. As used herein, the term "cannabinoid" refers to a class of diverse natural or synthetic chemical compounds that acts on cannabinoid receptors (i.e., CB1 and CB2) in cells that alter neurotransmitter release in the brain. Cannabinoids are cyclic molecules exhibiting particular properties such as the ability to easily cross the blood-brain barrier. Cannabinoids may be naturally occurring (Phytocannabinoids) from plants such as cannabis, (endocannabinoids) from animals, or artificially manufactured (synthetic cannabinoids). Cannabis species express at least 85 different phytocannabinoids, and these may be divided into subclasses, including cannabigerols, cannabichromenes, cannabidiols, tetrahydrocannabinols, cannabinols and cannabinodiols, and other cannabinoids, such as cannabigerol (CBG), cannabichromene (CBC), cannabidiol (CBD), tetrahydrocannabinol (THC), cannabinol (CBN) and cannabinodiol (CBDL), cannabicyclol (CBL), cannabivarin (CBV), tetrahydrocannabivarin (THCV), cannabidivarin (CBDV), cannabichromevarin (CBCV), cannabigerovarin (CBGV), cannabigerol monomethyl ether (CBGM), cannabinerolic acid, cannabidiolic acid (CBDA), Cannabinol propyl variant (CBNV), cannabitriol (CBO), tetrahydrocannabmolic acid (THCA), and tetrahydrocannabivarinic acid (THCV A).

In some embodiments, the cannabinoid is selected from the group consisting of cannabigerol (CBG), cannabichromene (CBC), cannabidiol (CBD), tetrahydrocannabinol (THC), cannabinol (CBN) and cannabinodiol (CBDL), cannabicyclol (CBL), cannabivarin (CBV), tetrahydrocannabivarin (THCV), cannabidivarin (CBDV), cannabichromevarin (CBCV), cannabigerovarin (CBGV), cannabigerol monomethyl ether (CBGM), cannabinerolic acid, cannabidiolic acid (CBDA), Cannabinol propyl variant (CBNV), cannabitriol (CBO), tetrahydrocannabmolic acid (THCA), tetrahydrocannabivarinic acid (THCV A), and mixtures thereof. In some embodiments, the cannabinoid comprises at least tetrahydrocannabinol (THC). In some embodiments, the cannabinoid is tetrahydrocannabinol (THC). In some embodiments, the cannabinoid comprises at least cannabidiol (CBD). In some embodiments, the cannabinoid is cannabidiol (CBD). In some embodiments, the CBD is synthetic CBD. Notably, CBD has a logP value of about 6.5, making it insoluble in an aqueous environment (e.g., saliva).

In some embodiments, the cannabinoid (e.g., CBD) is added to the composition in the form of an isolate. An isolate is an extract from a plant, such as cannabis, where the active material of interest (in this case the cannabinoid, such as CBD) is present in a high degree of purity, for example greater than 95%, greater than 96%, greater than 97%, greater than 98%, or around 99% purity.

In some embodiments, the cannabinoid is an isolate of CBD in a high degree of purity, and the amount of any other

cannabinoid in the effervescent composition is no greater than about 1% by weight of the oral product, such as no greater than about 0.5% by weight of the effervescent composition, such as no greater than about 0.1% by weight of the effervescent composition, such as no greater than about 0.01% by weight of the effervescent composition.

The choice of cannabinoid and the particular percentages thereof which may be present within the disclosed effervescent composition will vary depending upon the desired flavor, texture, and other characteristics of the effervescent composition.

When present, a cannabinoid (e.g., CBD) is typically in a concentration of at least about 0.1% by weight of the effervescent composition, such as in a range from about 0.1% to about 30%, such as, e.g., from about from about 0.1%, about 0.2%, about 0.3%, about 0.4%, about 0.5% about 0.6%, about 0.7%, about 0.8%, or about 0.9%, to about 1%, about 2%, about 3%, about 4%, about 5%, about 6%, about 7%, about 8%, about 9%, about 10%, about 15%, about 20%, or about 30% by weight, based on the total weight of the effervescent composition.

Alternatively, or in addition to the cannabinoid, the active agent may include a cannabimimetic, which is a class of compounds derived from plants other than cannabis that have biological effects on the endocannabinoid system similar to cannabinoids. Examples include yangonin, alpha-amyrin or beta-amyrin (also classified as terpenes), cyanidin, curcumin (tumeric), catechin, quercetin, salvinorin A, N-acylethanolamines, and N-alkylamide lipids. Such compounds can be used in the same amounts and ratios noted herein for cannabinoids.

***Terpenes***

Active ingredients suitable for use in the present disclosure can also be classified as terpenes, many of which are associated with biological effects, such as calming effects. Terpenes are understood to have the general formula of $(C5H8)n$ and include monoterpenes, sesquiterpenes, and diterpenes. Terpenes can be acyclic, monocyclic or bicyclic in structure. Some terpenes provide an entourage effect when used in combination with cannabinoids or cannabimimetics. Examples include beta-caryophyllene, linalool, limonene, beta-citronellol, linalyl acetate, pinene (alpha or beta), geraniol, carvone, eucalyptol, menthone, iso-menthone, piperitone, myrcene, beta-bourbonene, and germacrene, which may be used singly or in combination.

In some embodiments, the terpene is a terpene derivable from a phytocannabinoid producing plant, such as a plant from the stain of the cannabis sativa species, such as hemp. Suitable terpenes in this regard include so-called "C10" terpenes, which are those terpenes comprising 10 carbon atoms, and so-called "C15" terpenes, which are those terpenes comprising 15 carbon atoms. In some embodiments, the active ingredient comprises more than one terpene. For example, the active ingredient may comprise one, two, three, four, five, six, seven, eight, nine, ten or more terpenes as defined herein. In some embodiments, the terpene is selected from pinene (alpha and beta), geraniol, linalool, limonene, carvone, eucalyptol, menthone, iso-menthone, piperitone, myrcene, beta-bourbonene, germacrene and mixtures thereof.

***Pharmaceutical ingredients***

In some embodiments, the active ingredient comprises a pharmaceutical ingredient. The pharmaceutical ingredient can be any known agent adapted for therapeutic, prophylactic, or diagnostic use. These can include, for example, synthetic organic compounds, proteins and peptides, polysaccharides and other sugars, lipids, phospholipids, inorganic compounds (e.g., magnesium, selenium, zinc, nitrate), neurotransmitters or precursors thereof (e.g., serotonin, 5-hydroxy-tryptophan, oxitriptan, acetylcholine, dopamine, melatonin), and nucleic acid sequences, having therapeutic, prophylactic, or diagnostic activity. Non-limiting examples of pharmaceutical ingredients include analgesics and antipyretics (e.g., acetylsalicylic acid, acetaminophen, 3-(4-isobutylphenyl)propanoic acid), phosphatidylserine, myoinositol, docosahexaenoic acid (DHA, Omega-3), arachidonic acid (AA, Omega-6), S-adenosylmethionine (SAM), beta-hydroxy-beta-methylbutyrate (HMB), citicoline (cytidine-5'-diphosphate-choline), and cotinine.

The amount of pharmaceutical ingredient may vary. For example, when present, a pharmaceutical ingredient is typically at a concentration of from about 0.001% w/w to about 10% by weight, such as, e.g., from about from about 0.01%, about 0.02%, about 0.03%, about 0.04%, about 0.05%, about 0.06%, about 0.07%, about 0.08%, about 0.09%, about 0.1% w/w, about 0.2%, about 0.3%, about 0.4%, about 0.5% about 0.6%, about 0.7%, about 0.8%, about 0.9%, or about 1%, to about 2%, about 3%, about 4%, about 5%, about 6%, about 7%, about 8%, about 9%, or about 10% by weight, based on the total weight of the effervescent composition.

***Sensates***

In some embodiments, the effervescent composition comprises a sensate (e.g., as an active ingredient, a component of the effervescent composition, or both). Sensates are compounds which chemically induce somatosensorial sensation through stimulation of the fifth cranial nerve (trigeminal nerve), in addition to or in place of aroma or taste nerves. Sensates include agents providing warming/heating, cooling, tingling, and numbing effects by interacting with (e.g.,

stimulating) the trigeminal nerve. For avoidance of doubt, such sensates are different and distinct from the flavoring agents disclosed herein.

Suitable sensates which provide to the user a warming effect include, but are not limited to, vanillyl alcohol n-butyl ether, vanillyl alcohol n-propylether, vanillyl alcohol isopropyl ether, vanillyl alcohol isobutyl ether, vanillyl alcohol n-aminoether, vanillyl alcohol isoamyl ether, vanillyl alcohol n-hexyl ether, vanillyl alcohol methyl ether, vanillyl alcohol ethyl ether, gingerol, shogaol, paradol, zingerone, capsaicin, dihydrocapsaicin, nordihydrocapsaicin, homocapsaicin, homodihydrocapsaicin, ethanol, isopropyl alcohol, iso-amylalcohol, benzyl alcohol, glycerin, and combinations thereof. In some embodiments, the warming agent comprises vanillyl ethyl ether, capsaicin, or a combination thereof.

Suitable sensates which provide to the user a cooling effect include, but are not limited to, menthane, menthone, menthone ketals, menthone glycerol ketals, substituted p-menthanes, acyclic carboxamides, monomenthyl glutarate, substituted cyclohexanamides, substituted cyclohexane carboxamides, substituted ureas and sulfonamides, substituted menthanols, hydroxymethyl and hydroxymethyl derivatives of p-menthane, 2-mercapto-cyclo-decanone, hydroxycarboxylic acids with 2-6 carbon atoms, cyclohexanamides, menthyl acetate, menthyl salicylate, N,2,3-trimethyl-2-isopropyl butanamide (WS-23), N-ethyl-2,2-diisopropylbutanamide, N-ethyl-p-menthane-3-carboxamide (WS-3), ethyl ester of N-[[5-methyl-2-(1-methylethyl)cyclohexyl]carbonyl]glycine (WS5), as well as the substantially pure ethyl ester of N-[[5-methyl-2-(1-methylethyl)cyclohexyl]carbonyl]glycine as disclosed in U.S. Pat. No. 7,189,760 to Erman, et al which is incorporated in its entirety herein by reference, isopulegol, menthyloxy propane diol, 3-(1-menthoxy)propane-1,2-diol, 3-(1-menthoxy)-2-methylpropane-1,2-diol, p-menthane-2,3-diol, p-menthane-3,8-diol, 6-isopropyl-9-methyl-1,4-dioxaspiro[4,5]decane-2-methanol, menthyl succinate and its alkaline earth metal salts, trimethylcyclohexanol, N-ethyl-2-isopropyl-5-methylcyclohexanecarboxamide, Japanese mint oil, peppermint oil, 3-(1-menthoxy)ethan-1-ol, 3-(1-menthoxy)propan-1-ol, 3-(1-menthoxy)butan-1-ol, 1-menthylacetic acid N-ethylamide, 1-menthyl-4-hydroxypentanoate, 1-menthyl-3-hydroxybutyrate, N,2,3-trimethyl-2-(1-methylethyl)-butanamide, n-ethyl-t-2-c-6 nonadienamide, N,N-dimethyl menthyl succinamide, substituted p-menthanes, substituted p-menthane-carboxamides, 2-isopropanyl-5-methylcyclohexanol; menthone glycerol ketals; 3-1-menthoxypropane-1,2-diol; and menthyl lactate; (from Haarman & Reimer, FEMA 3748, tradename FRESCOLAT.RTM. type ML), WS-30, WS-14, Eucalyptus extract (p-Mehtha-3,8-Diol), Menthol (its natural or synthetic derivatives), Menthol PG carbonate, Menthol EG carbonate, Menthol glyceryl ether, N-tertbutyl-p-menthane-3-carboxamide, P-menthane-3-carboxylic acid glycerol ester, Methyl-2-isopryl-bicyclo (2.2.1), Heptane-2-carboxamide; Menthol methyl ether, menthyl pyrrolidone carboxylate; 2,5-dimethyl-4-(1-pyrrolidinyl)-3(2H)-furanone; cyclic a-keto enamines, cyclotene derivatives such as cyclopentenes including 3-methyl-2-(1-pyrrolidinyl)-2-cyclopenten-1-one and 5-methyl-2-(1-pyrrolidinyl)-2-cyclopenten-1-one. Other compounds include the alpha-keto enamines disclosed in U.S. Pat. No. 6,592,884 to Hofmann et al., which is incorporated in its entirety herein. These and other suitable cooling agents are further described in the following U.S. patents, all of which are incorporated in their entirety by reference hereto: U.S. Pat. No. 4,230,688; 4,032,661; 4,459,425; 4,178,459; 4,296,255; 4,136,163; 5,009,893; 5,266,592; 5,698,181; 6,277,385; 6,627,233; 7,030,273. Still other suitable cooling agents are further described in the following U.S. Patent Applications, all of which are incorporated in their entirety by reference hereto: U.S. 2005/0222256; 2005/0265930. In some embodiments, the cooling agent comprises menthol, eucalyptus, mint, menthol, menthyl esters, eucolyptol, WS-3, WS-23, WS-5, EvercoolTM 180 ((1R,2S,5R)-N-(4-(cyanomethyl)phenyl)menthylcarboxamide), EvercoolTM 190 ((1R,2S,SR)-N-(2-(pyridin-2-yl)ethyl)menthylcarboxamide), or a combination thereof.

Suitable sensates which provide to the user a tingling, stinging, or numbing effect include, but are not limited to Jambu Oleoresin or para cress (Spilanthes sp.), in which the active ingredient is Spilanthol; Japanese pepper extract (Zanthoxylum peperitum), including the ingredients known as Saanshool-I, Saanshool-II and Sanshoamide; perillartine; 4-(1-menthoxymethyl)-2-phenyl-1,3-dioxolane; black pepper extract (piper nigrum), including the active ingredients chavicine and piperine; Echinacea extract; Northern Prickly Ash extract; trans-pellitorin, and red pepper oleoresin. In some embodiments, alkylamides extracted from materials such as jambu or sanshool may be included. Further examples of "tingling" type sensates include those disclosed in U.S. Pat. Nos. 6,780,443, 6,159,509, 5,545,424, and 5,407,665, each of which is incorporated by reference herein in its entirety.

### *Ion Pairing of Basic Amine-Containing Active Ingredients and Organic Acids*

In some embodiments, the active ingredient has a basic amine functionality (i.e., the active ingredient comprises a basic amine). By "basic amine" is meant a molecule including at least one basic amine functional group. Examples of basic amines include, but are not limited to, alkaloids. By "basic amine functional group" is meant a group containing a nitrogen atom having a lone pair of electrons. The basic amine functional group is attached to or incorporated within the molecule through one or more covalent bonds to the said nitrogen atom. The basic amine may be a primary, secondary, or tertiary amine, meaning the nitrogen bears one, two, or three covalent bonds to carbon atoms. By virtue of the lone pair of electrons on the nitrogen atom, such amines are termed "basic", meaning the lone electron pair is available for hydrogen bonding. The basicity (i.e., the electron density on the nitrogen atom and consequently the availability and strength of hydrogen bonding to the nitrogen atom) of the basic amine may be influenced by the nature of neighboring atoms, the steric

bulk of the molecule, and the like.

Generally, the basic amine is released from the composition and absorbed through the oral mucosa, thereby entering the blood stream, where it is circulated systemically. Generally, the basic amine is present in or as an active ingredient in the composition, as described herein below.

In some embodiments, the active ingredient having the basic amine functionality is caffeine. In some embodiments, the active ingredient having a basic amine functionality is nicotine, for example, a nicotine component as described herein above.

In some embodiments, at least a portion the active ingredient having the basic amine functionality (e.g., nicotine) is associated with at least a portion of the organic acid or the alkali metal salt thereof which may be present in some embodiments. Depending on multiple variables (concentration, pH, nature of the organic acid, and the like), the active ingredient having the basic amine functionality present in the composition can exist in multiple forms, including ion paired, in solution (i.e., fully solvated), as the free base, as a cation, as a salt, or any combination thereof.

In some embodiments, at least a portion of the active ingredient present in the composition and having the basic amine functionality is in an ion paired form with an organic acid or conjugate base thereof as further described herein below. In some embodiments, the active ingredient having the basic amine functionality and at least a portion of the organic acid or the alkali metal salt thereof is in the form of an ion pair between the basic amine and a conjugate base of the organic acid.

Ion pairing describes the partial association of oppositely charged ions in relatively concentrated solutions to form distinct chemical species called ion pairs. The strength of the association (i.e., the ion pairing) depends on the electrostatic force of attraction between the positive and negative ions (i.e., a protonated basic amine and the conjugate base of an organic acid). By "conjugate base" is meant the base resulting from deprotonation of the corresponding acid (e.g., benzoate is the conjugate base of benzoic acid).

On average, a certain population of these ion pairs exists at any given time, although the formation and dissociation of ion pairs is continuous. In the composition as disclosed herein, and/or upon oral use of said composition (e.g., upon contact with saliva), the active ingredient having the basic amine functionality and the conjugate base of the organic acid exist at least partially in the form of an ion pair. Without wishing to be bound by theory, it is believed that such ion pairing may minimize chemical degradation of the active ingredient having the basic amine functionality and/or enhance the oral availability of the active ingredient having the basic amine functionality. At alkaline pH values (e.g., such as from about 7.5 to about 9), certain active ingredient having the basic amine functionality, for example nicotine, are largely present in the free base form, which has relatively low water solubility, and low stability with respect to evaporation and oxidative decomposition, but high mucosal availability. Conversely, at acidic pH values (such as from about 6.5 to about 4), certain active ingredient having the basic amine functionality, for example nicotine, are largely present in a protonated form, which has relatively high solubility in water, and higher stability with respect to evaporation and oxidative decomposition, but low mucosal availability. In some embodiments, the properties of stability, solubility, and availability of nicotine can be enhanced through ion pairing or salt formation of nicotine with appropriate organic acids and/or their conjugate bases. Specifically, nicotine-organic acid ion pairs of moderate lipophilicity result in favorable stability and absorption properties. Lipophilicity is conveniently measured in terms of logP, the partition coefficient of a molecule between a lipophilic phase and an aqueous phase, usually octanol and water, respectively. An octanol-water partitioning favoring distribution of an ion pair into octanol is predictive of good absorption of the active ingredient having the basic amine functionality present in the composition through the oral mucosa.

As noted above, at alkaline pH values (e.g., such as from about 7.5 to about 9), nicotine is largely present in the free base form (and accordingly, a high partitioning into octanol), while, at acidic pH values (such as from about 6.5 to about 4), nicotine is largely present in a protonated form (and accordingly, a low partitioning into octanol). Surprisingly, according to the present disclosure, it has been found that an ion pair between certain organic acids (e.g., having a logP value of from about 1.4 to about 8.0. such as from about 1.4 to about 4.5, allows nicotine partitioning into octanol consistent with that predicted for nicotine partitioning into octanol at a pH of 8.4.

One of skill in the art will recognize that the extent of ion pairing in the disclosed composition, both before and during use by the consumer, may vary based on, for example, pH, the nature of the organic acid, the concentration of nicotine, the concentration of the organic acid or conjugate base of the organic acid present in the composition, the moisture content of the composition, the ionic strength of the composition, and the like. One of skill in the art will also recognize that ion pairing is an equilibrium process influenced by the foregoing variables. Accordingly, quantification of the extent of ion pairing is difficult or impossible by calculation or direct observation. However, the presence of ion pairing may be demonstrated through surrogate measures such as partitioning between octanol and water or membrane permeation of aqueous solutions of nicotine plus organic acids and/or their conjugate bases.

*Organic acid*

As discussed herein above, in some embodiments, the effervescent composition as disclosed herein comprises an organic acid, an alkali metal salt thereof, or both. In some embodiments, at least a portion of the organic acid or salt

thereof is in the form of an ion pair with a basic amine-containing active ingredient as described herein above.

As used herein, the term "organic acid" refers to an organic (i.e., carbon-based) compound that is characterized by acidic properties. Typically, organic acids are relatively weak acids (i.e., they do not dissociate completely in the presence of water), such as carboxylic acids (-CO2H) or sulfonic acids (-SO2OH). As used herein, reference to organic acid means an organic acid that is intentionally added. In this regard, an organic acid may be intentionally added as a specific composition ingredient as opposed to merely being inherently present as a component of another composition ingredient (e.g., the small amount of organic acid which may inherently be present in a composition ingredient, such as a tobacco material).

Suitable organic acids will typically have a range of lipophilicities (i.e., a polarity giving an appropriate balance of water and organic solubility). Typically, lipophilicities of suitable organic acids, as indicated by logP, will vary between about 1 and about 12 (more soluble in octanol than in water). In some embodiments, the organic acid has a logP value from about 1 to about 12, e.g., from about 1.0. about 1.5, about 2.0, about 2.5, about 3.0, about 3.5, about 4.0, about 4.5, about 5.0, about 5.5, about 6.0, about 6.5, about 7.0, about 7.5, or about 8.0, to about 8.5, about 9.0, about 9.5, about 10.0, about 10.5, about 11.0, about 11.5, or about 12.0.

Without wishing to be bound by theory, it is believed that moderately lipophilic organic acids (e.g., logP of from about 1.4 to about 4.5) produce ion pairs with nicotine which are of a polarity providing good octanol-water partitioning of the ion pair, and hence partitioning of nicotine, into octanol versus water. As discussed above, such partitioning into octanol is predictive of favorable oral availability.

In some embodiments, the organic acid has a logP value from about 3.0 to about 8.0, about 10.0, or even 12.0. In some embodiments, the presence of certain solvents or solubilizing agents (e.g., inclusion in the composition of glycerin or propylene glycol) may be beneficial in solubilizing organic acids and the corresponding salts or ion pairs thereof with the basic amine for highly lipophilic organic acids (e.g., higher than about 4.5).

In some embodiments, the organic acid is a carboxylic acid or a sulfonic acid. The carboxylic acid or sulfonic acid functional group may be attached to any alkyl, cycloalkyl, heterocycloalkyl, aryl, or heteroaryl group having, for example, from one to twenty carbon atoms (C1-C20). In some embodiments, the organic acid is an alkyl, cycloalkyl, heterocycloalkyl, aryl, or heteroaryl carboxylic or sulfonic acid.

As used herein, "alkyl" refers to any straight chain or branched chain hydrocarbon. The alkyl group may be saturated (i.e., having all sp3 carbon atoms), or may be unsaturated (i.e., having at least one site of unsaturation). As used herein, the term "unsaturated" refers to the presence of a carbon-carbon, sp2 double bond in one or more positions within the alkyl group. Unsaturated alkyl groups may be mono- or polyunsaturated. Representative straight chain alkyl groups include, but are not limited to, methyl, ethyl, n-propyl, n-butyl, n-pentyl, and n-hexyl. Branched chain alkyl groups include, but are not limited to, isopropyl, sec-butyl, isobutyl, tert-butyl, isopentyl, and 2-methylbutyl. Representative unsaturated alkyl groups include, but are not limited to, ethylene or vinyl, allyl, 1-butenyl, 2-butenyl, isobutylenyl, 1-pentenyl, 2-pentenyl, 3-methyl-1-butenyl, 2-methyl-2-butenyl, 2,3-dimethyl-2-butenyl, and the like. An alkyl group can be unsubstituted or substituted.

"Cycloalkyl" as used herein refers to a carbocyclic group, which may be mono- or bicyclic. Cycloalkyl groups include rings having 3 to 7 carbon atoms as a monocycle or 7 to 12 carbon atoms as a bicycle. Examples of monocyclic cycloalkyl groups include cyclopropyl, cyclobutyl, cyclopentyl, cyclohexyl, cycloheptyl, and cyclooctyl. A cycloalkyl group can be unsubstituted or substituted, and may include one or more sites of unsaturation (e.g., cyclopentenyl or cyclohexenyl).

The term "aryl" as used herein refers to a carbocyclic aromatic group. Examples of aryl groups include, but are not limited to, phenyl and naphthyl. An aryl group can be unsubstituted or substituted.

"Heteroaryl" and "heterocycloalkyl" as used herein refer to an aromatic or non-aromatic ring system, respectively, in which one or more ring atoms is a heteroatom, e.g. nitrogen, oxygen, and sulfur. The heteroaryl or heterocycloalkyl group comprises up to 20 carbon atoms and from 1 to 3 heteroatoms selected from N, O, and S. A heteroaryl or heterocycloalkyl may be a monocycle having 3 to 7 ring members (for example, 2 to 6 carbon atoms and 1 to 3 heteroatoms selected from N, O, and S) or a bicycle having 7 to 10 ring members (for example, 4 to 9 carbon atoms and 1 to 3 heteroatoms selected from N, O, and S), for example: a bicyclo[4,5], [5,5], [5,6], or [6,6] system. Examples of heteroaryl groups include by way of example and not limitation, pyridyl, thiazolyl, tetrahydrothiophenyl, pyrimidinyl, furanyl, thienyl, pyrrolyl, pyrazolyl, imidazolyl, tetrazolyl, benzofuranyl, thianaphthalenyl, indolyl, indolenyl, quinolinyl, isoquinolinyl, benzimidazolyl, isoxazolyl, pyrazinyl, pyridazinyl, indolizinyl, isoindolyl, 3H-indolyl, 1H-indazolyl, purinyl, 4H-quinolizinyl, phthalazinyl, naphthyridinyl, quinoxalinyl, quinazolinyl, cinnolinyl, pteridinyl, 4aH-carbazolyl, carbazolyl, phenanthridinyl, acridinyl, pyrimidinyl, phenanthrolinyl, phenazinyl, phenothiazinyl, furazanyl, phenoxazinyl, isochromanyl, chromanyl, imidazolidinyl, imidazolinyl, pyrazolidinyl, pyrazolinyl, benzotriazolyl, benzisoxazolyl, and isatinoyl. Examples of heterocycloalkyls include by way of example and not limitation, dihydroypyridyl, tetrahydropyridyl (piperidyl), tetrahydrothiophenyl, piperidinyl, 4-piperidonyl, pyrrolidinyl, 2-pyrrolidonyl, tetrahydrofuranyl, tetrahydropyranyl, bis-tetrahydropyranyl, tetrahydroquinolinyl, tetrahydroisoquinolinyl, decahydroquinolinyl, octahydroisoquinolinyl, piperazinyl, quinuclidinyl, and morpholinyl. Heteroaryl and heterocycloalkyl groups can be unsubstituted or substituted.

"Substituted" as used herein and as applied to any of the above alkyl, aryl, cycloalkyl, heteroaryl, heterocyclyl, means that one or more hydrogen atoms are each independently replaced with a substituent. Typical substituents include, but are not limited to, -Cl, Br, F, alkyl, -OH, - OCH3, NH2, -NHCH3, -N(CH3)2, -CN, -NC(=O)CH3, -C(=O)-, -C(=O)NH2, and - C(=O)N(CH3)2. Wherever a group is described as "optionally substituted," that group can be substituted with one or more of the above substituents, independently selected for each occasion. In some embodiments, the substituent may be one or more methyl groups or one or more hydroxyl groups.

In some embodiments, the organic acid is an alkyl carboxylic acid. Non-limiting examples of alkyl carboxylic acids include formic acid, acetic acid, propionic acid, butyric acid, valeric acid, caproic acid, heptanoic acid, octanoic acid, nonanoic acid, decanoic acid, undecanoic acid, dodecanoic acid, stearic acid, oleic acid, linoleic acid, linolenic acid, and the like.

In some embodiments, the organic acid is an alkyl sulfonic acid. Non-limiting examples of alkyl sulfonic acids include propanesulfonic acid, heptanesulfonic acid, and octanesulfonic acid.

In some embodiments, the alkyl carboxylic or sulfonic acid is substituted with one or more hydroxyl groups. Non-limiting examples include glycolic acid, 4-hydroxybutyric acid, and lactic acid.

In some embodiments, an organic acid may include more than one carboxylic acid group or more than one sulfonic acid group (e.g., two, three, or more carboxylic acid groups). Non-limiting examples include oxalic acid, fumaric acid, maleic acid, and glutaric acid. In organic acids containing multiple carboxylic acids (e.g., from two to four carboxylic acid groups), one or more of the carboxylic acid groups may be esterified. Non-limiting examples include succinic acid monoethyl ester, monomethyl fumarate, monomethyl or dimethyl citrate, and the like.

In some embodiments, the organic acid may include more than one carboxylic acid group and one or more hydroxyl groups. Non-limiting examples of such acids include tartaric acid, citric acid, and the like.

In some embodiments, the organic acid is an aryl carboxylic acid or an aryl sulfonic acid. Non-limiting examples of aryl carboxylic and sulfonic acids include benzoic acid, toluic acids, salicylic acid, benzenesulfonic acid, and p-toluenesulfonic acid.

Further non-limiting examples of organic acids which may be useful in some embodiments include dibenzoyl-L-tartaric acid, 2,2-dichloroacetic acid, 2-hydroxyethanesulfonic acid, 2-oxoglutaric acid, 4-acetamidobenzoic acid, 4-aminosalicylic acid, adipic acid, ascorbic acid (L), aspartic acid (L), alpha-methylbutyric acid, camphoric acid (+), camphor-10-sulfonic acid (+), cinnamic acid, cyclamic acid, dodecylsulfuric acid, ethane-1,2-disulfonic acid, ethanesulfonic acid, furoic acid, galactaric acid, gentisic acid, glucoheptonic acid, gluconic acid, glucuronic acid, glutamic acid, glycerophosphoric acid, glycolic acid, hippuric acid, isobutyric acid, isovaleric acid, lactobionic acid, lauric acid, levulinic acid, malic acid, malonic acid, mandelic acid, methanesulfonic acid, naphthalene-1,5-disulfonic acid, naphthalene-2-sulfonic acid, oleic acid, palmitic acid, pamoic acid, phenylacetic acid, pyroglutamic acid, pyruvic acid, sebacic acid, stearic acid, and undecylenic acid. Examples of suitable acids include, but are not limited to, the list of organic acids in Table 1.

Table 1. Non-limiting examples of suitable organic acids

| Acid Name | log(P)* |
|---|---|
| benzoic acid | 1.9 |
| phenylacetic | 1.4 |
| p-toluic acid | 2.3 |
| ethyl benzoic acid | 2.9 |
| isopropyl benzoic acid | 3.5 |
| 4-phenylbutyric | 2.4 |
| 2-(4-Isobutylphenyl)propanoic acid | 3.5 |
| 2-napthoxyacetic acid | 2.5 |
| napthylacetic acid | 2.7 |
| heptanoic acid | 2.5 |
| octanoic acid | 3.05 |
| nonanoic acid | 3.5 |
| decanoic acid | 4.09 |
| 9-deceneoic acid | 3.3 |
| 2-deceneoic acid | 3.8 |

(continued)

| Acid Name | log(P)* |
|---|---|
| 10-undecenoic acid | 3.9 |
| dodecandioic acid | 3.2 |
| dodecanoic acid | 4.6 |
| myristic acid | 5.3 |
| palmitic acid | 6.4 |
| stearic acid | 7.6 |
| cyclohexanebutanoic acid | 3.4 |
| 1-heptanesulfonic acid | 2.0 |
| 1-octanesulfonic acid | 2.5 |
| 1-nonanesulfonic acid | 3.1 |
| monooctyl succinate | 2.8 |
| tocopherol succinate | 10.2 |
| monomenthyl succinate | 3 |
| monomenthyl glutarate | 3.4 |
| norbixin ((2E,4E,6E,8E,10E,12E,14E,16E,18E)-4,8,13,17-tetramethylicosa-2,4,6,8,10,12,14,16,18-nonaenedioic acid) | 7.2 |
| bixin ((2E,4E,6E,8E,10E,12E,14E,16Z,18E)-20-methoxy-4,8,13,17-tetramethyl-20-oxoicosa-2,4,6,8,10,12,14,16,18-nonaenoic acid) | 7.5 |
| *Values obtained from PubChem or calculated | |

The selection of organic acid may further depend on additional properties in addition to consideration of the logP value. For example, an organic acid should be one recognized as safe for human consumption, and which has acceptable flavor, odor, volatility, stability, and the like. Determination of appropriate organic acids is within the purview of one of skill in the art.

In some embodiments, the organic acid is a mono ester of a dicarboxylic acid or a polycarboxylic acid. In some embodiments, the dicarboxylic acid is malonic acid, succinic acid, glutaric acid, adipic acid, fumaric acid, maleic acid, or a combination thereof. In some embodiments, the dicarboxylic acid is succinic acid, glutaric acid, fumaric acid, maleic acid, or a combination thereof. In some embodiments, the dicarboxylic acid is succinic acid, glutaric acid, or a combination thereof.

In some embodiments, the alcohol forming the mono ester of the dicarboxylic acid is a lipophilic alcohol. Examples of suitable lipophilic alcohols include, but are not limited to, octanol, menthol, and tocopherol. In some embodiments, the organic acid is an octyl mono ester of a dicarboxylic acid, such as monooctyl succinate, monooctyl fumarate, or the like. In some embodiments, the organic acid is a monomenthyl ester of a dicarboxylic acid. Certain menthyl esters may be desirable in effervescent composition as described herein by virtue of the cooling sensation they may provide upon use of the product comprising the composition. In some embodiments, the organic acid is monomenthyl succinate, monomenthyl fumarate, monomenthyl glutarate, or a combination thereof. In some embodiments, the organic acid is a monotocopheryl ester of a dicarboxylic acid. Certain tocopheryl esters may be desirable in effervescent composition as described herein by virtue of the antioxidant effects they may provide. In some embodiments, the organic acid is tocopheryl succinate, tocopheryl fumarate, tocopheryl glutarate, or a combination thereof.

In some embodiments, the organic acid is a carotenoid derivative having one or more carboxylic acids. Carotenoids are tetraterpenes, meaning that they are produced from 8 isoprene molecules and contain 40 carbon atoms. Accordingly, they are usually lipophilic due to the presence of long unsaturated aliphatic chains, and are generally yellow, orange, or red in color. Certain carotenoid derivatives can be advantageous in effervescent composition by virtue of providing both ion pairing and serving as a colorant in the composition. In some embodiments, the organic acid is 2E,4E,6E,8E,10E,12E,14E,16Z,18E)-20-methoxy-4,8,13,17-tetramethyl-20-oxoicosa-2,4,6,8,10,12,14,16,18-nonaenoic acid (bixin) or an isomer thereof. Bixin is an apocarotenoid found in annatto seeds from the achiote tree (Bixa orellana) and is the naturally occurring pigment providing the reddish orange color to annatto. Bixin is soluble in fats and alcohols but insoluble in water, and is chemically unstable when isolated, converting via isomerization into the double bond isomer,

trans-bixin (β-bixin), having the structure:

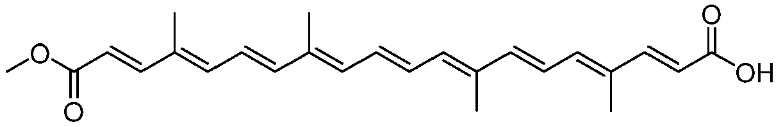

.

In some embodiments, the organic acid is (2E,4E,6E,8E,10E,12E,14E,16E,18E)-4,8,13,17-tetramethylicosa-2,4,6,8,10,12,14,16,18-nonaenedioic acid (norbixin), a water-soluble hydrolysis product of bixin having the structure:

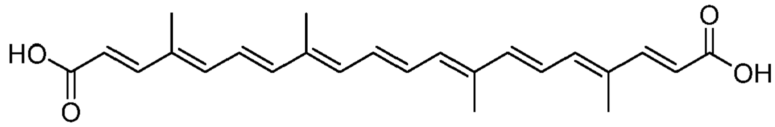

.

The selection of organic acid may further depend on additional properties in addition to or without consideration to the logP value. For example, an organic acid should be one recognized as safe for human consumption, and which has acceptable flavor, odor, volatility, stability, and the like. Determination of appropriate organic acids is within the purview of one of skill in the art.

In some embodiments, more than one organic acid may be present. For example, the composition may comprise two, or three, or four, or more organic acids. Accordingly, reference herein to "an organic acid" contemplates mixtures of two or more organic acids. The relative amounts of the multiple organic acids may vary. For example, a composition may comprise equal amounts of two, or three, or more organic acids, or may comprise different relative amounts. In this manner, it is possible to include certain organic acids (e.g., citric acid or myristic acid) which have a logP value outside the desired range, when combined with other organic acids to provide the desired average logP range for the combination. In some embodiments, it may be desirable to include organic acids in the composition which have logP values outside the desired range for purposes such as, but not limited to, providing desirable organoleptic properties, stability, as flavor components, and the like. Further, certain lipophilic organic acids have undesirable flavor and or aroma characteristics which would preclude their presence as the sole organic acid (e.g., in equimolar or greater quantities relative to nicotine). Without wishing to be bound by theory, it is believed that a combination of different organic acids may provide the desired ion pairing while the concentration of any single organic acid in the composition remains below the threshold which would be found objectionable from a sensory perspective.

For example, in some embodiments, the organic acid may comprise from about 1 to about 5 or more molar equivalents of benzoic acid relative to nicotine, combined with e.g., about 0.2 molar equivalents of octanoic acid or a salt thereof, and 0.2 molar equivalents of decanoic acid or a salt thereof.

In some embodiments, the organic acid is a combination of any two organic acids selected from the group consisting of benzoic acid, a toluic acid, benzenesulfonic acid, toluenesulfonic acid, hexanoic acid, heptanoic acid, decanoic acid, and octanoic acid. In some embodiments, the organic acid is a combination of benzoic acid, octanoic acid, and decanoic acid, or benzoic and octanoic acid. In some embodiments, the composition comprises citric acid in addition to one or more of benzoic acid, a toluic acid, benzenesulfonic acid, toluenesulfonic acid, hexanoic acid, heptanoic acid, decanoic acid, and octanoic acid.

In some embodiments, the effervescent composition comprises an alkali metal salt of an organic acid. For example, at least a portion of the organic acid may be present in the composition in the form of an alkali metal salt. Suitable alkali metal salts include lithium, sodium, and potassium. In some embodiments, the alkali metal is sodium or potassium. In some embodiments, the alkali metal is sodium. In some embodiments, the composition comprises an organic acid and a sodium salt of the organic acid.

In some embodiments, the effervescent composition comprises benzoic acid and sodium benzoate, octanoic acid and sodium octanoate, decanoic acid and sodium decanoate, or a combination thereof. In some embodiments, the composition comprises benzoic acid and sodium benzoate. In some embodiments, the composition comprises sodium benzoate.

In some embodiments, the ratio of the organic acid to the sodium salt (or other alkali metal salt) of the organic acid is from about 0.1 to about 10, such as from about 0.1, about 0.25, about 0.3, about 0.5, about 0.75, or about 1, to about 2, about 5, or about 10. For example, in some embodiments, both an organic acid and the sodium salt thereof are added to the other components of the composition, wherein the organic acid is added in excess of the sodium salt, in equimolar quantities with the sodium salt, or as a fraction of the sodium salt. One of skill in the art will recognize that the relative amounts will be determined by the desired pH of the composition, as well as the desired ionic strength. For example, the organic acid may be added in a quantity to provide a desired pH level of the composition, while the alkali metal (e.g.,

sodium) salt is added in a quantity to provide the desired extent of ion pairing. As one of skill in the art will understand, the quantity of organic acid (i.e., the protonated form) present in the composition, relative to the alkali metal salt or conjugate base form present in the composition, will vary according to the pH of the composition and the pKa of the organic acid, as well as according to the actual relative quantities initially added to the composition.

The amount of organic acid or an alkali metal salt thereof present in the effervescent composition, relative to the basic amine containing active ingredient (e.g., nicotine), may vary. Generally, as the concentration of the organic acid (or the conjugate base thereof) increases, the percent of nicotine that is ion paired with the organic acid increases. This typically increases the partitioning of the nicotine, in the form of an ion pair, into octanol versus water as measured by the logP (the log10 of the partitioning coefficient). In some embodiments, the composition comprises from about 0.05, about 0.1, about 1, about 1.5, about 2, or about 5, to about 10, about 15, or about 20 molar equivalents of the organic acid, the alkali metal salt thereof, or the combination thereof, relative to the nicotine component, calculated as free base nicotine.

In some embodiments, the effervescent composition comprises from about 2 to about 10, or from about 2 to about 5 molar equivalents of the organic acid, the alkali metal salt thereof, or the combination thereof, to nicotine, on a free-base nicotine basis. In some embodiments, the organic acid, the alkali metal salt thereof, or the combination thereof, is present in a molar ratio with the nicotine from about 2, about 3, about 4, or about 5, to about 6, about 7, about 8, about 9, or about 10. In embodiments wherein more than one organic acid, alkali metal salt thereof, or both, are present, it is to be understood that such molar ratios reflect the totality of the organic acids present.

In some embodiments the organic acid inclusion is sufficient to provide a pH of the effervescent composition from about 4.0 to about 9.5, such as from about 4.0 to about 9.0, or from about 4.0 to about 8.5, or from about 4.0 to about 8.0, or from about 4.5 to about 7.5, or from about 4.5 to about 7.0, or from about 5.5 to about 7.0, or from about 4.0 to about 5.5, or from about 7.0 to about 9.5. In some embodiments, the organic acid inclusion is sufficient to provide a composition pH of about 4.0, about 4.5, about 5.0, about 5.5, about 6.0, about 6.5, about 7.0, about 7.5, about 8.0, about 8.5, or about 9.0. In some embodiments, the organic acid inclusion is sufficient to provide a composition pH of from about 4.5 to about 6.5, for example, from about 4.5, about 5.0, or about 5.5, to about 6.0, or about 6.5. In some embodiments, the organic acid is provided in a quantity sufficient to provide a pH of the composition of from about 5.5 to about 6.5, for example, from about 5.5, about 5.6, about 5.7, about 5.8, about 5.9, or about 6.0, to about 6.1, about 6.2, about 6.3, about 6.4, or about 6.5. In other embodiments, a mineral acid (e.g., hydrochloric acid, sulfuric acid, phosphoric acid, or the like) is added to adjust the pH of the composition to the desired value.

In some embodiments, the organic acid is added as the free acid, either neat (i.e., native solid or liquid form) or as a solution in, e.g., water, to the other effervescent composition components. In some embodiments, the alkali metal salt of the organic acid is added, either neat or as a solution in, e.g., water, to the other effervescent composition components.

In some embodiments, the organic acid is added as the free acid, either neat (i.e., native solid or liquid form) or as a solution in, e.g., water, to the other composition components. In some embodiments, the alkali metal salt of the organic acid is added, either neat or as a solution in, e.g., water, to the other composition components. In some embodiments, the organic acid and the basic amine (e.g., nicotine) are combined to form a salt, either before addition to the composition, or the salt is formed within and is present in the composition as such. In other embodiments, the organic acid and basic amine (e.g., nicotine) are present as individual components in the composition and form an ion pair upon contact with moisture (e.g., saliva in the mouth of the consumer).

In some embodiments, the effervescent composition comprises nicotine benzoate and sodium benzoate, wherein at least a portion of the nicotine and benzoate ions present are in an ion paired form. In some embodiments, the composition comprises nicotine benzoate, sodium benzoate, and an organic acid, an alkali metal salt of an organic acid, or a combination thereof, the organic acid having a logP value from about 1 to about 12, wherein the organic acid is a monoester of a dicarboxylic acid or is a carotenoid derivative having one or more carboxylic acids.

In some embodiments, the effervescent composition further comprises a solubility enhancer to increase the solubility of one or more of the organic acid or salt thereof. Suitable solubility enhancers include, but are not limited to, humectants as described herein, such as glycerol or propylene glycol.

In some embodiments, the effervescent composition comprises nicotine benzoate and sodium benzoate. In some embodiments, the sodium benzoate is present in a molar ratio to the nicotine benzoate in a range from about 1 to about 20, such as about 2, about 5, or about 10. In some embodiments, the effervescent composition further comprises nicotine polacrilex. In some embodiments, the total amount of nicotine present in the composition is provided in equal quantities from nicotine benzoate and nicotine polacrilex. In some embodiments, the effervescent composition includes only nicotine polacrilex as the nicotine source, and the composition optionally comprises an organic acid component such as sodium benzoate.

### Flavoring agent

In some embodiments, the effervescent composition as described herein comprises a flavoring agent as an active ingredient, within the overall composition, or both. As used herein, a "flavoring agent" or "flavorant" is any flavorful or

aromatic substance capable of altering the sensory characteristics associated with the effervescent composition. Examples of sensory characteristics that can be modified by the flavoring agent include taste, mouthfeel, moistness, coolness/heat, and/or fragrance/aroma. Flavoring agents may be natural or synthetic, and the character of the flavors imparted thereby may be described, without limitation, as fresh, sweet, herbal, confectionary, floral, fruity, or spicy.

Flavoring agents may be imitation, synthetic or natural ingredients or blends thereof. Flavoring agents may include naturally occurring flavor materials, botanicals, extracts of botanicals, synthetically obtained materials, or combinations thereof (e.g., tobacco, cannabis, licorice (liquorice), hydrangea, Japanese white bark magnolia leaf, chamomile, fenugreek, maple, matcha, Japanese mint, aniseed (anise), turmeric, Indian spices, Asian spices, herb, wintergreen, cherry, berry, red berry, cranberry, peach, apple, orange, mango, clementine, lemon, lime, tropical fruit, papaya, rhubarb, grape, durian, dragon fruit, cucumber, blueberry, mulberry, citrus fruits, Drambuie, bourbon, scotch, whiskey, gin, tequila, rum, spearmint, peppermint, lavender, aloe vera, cardamom, celery, cascarilla, nutmeg, sandalwood, bergamot, geranium, khat, naswar, betel, shisha, pine, honey essence, rose oil, vanilla, lemon oil, orange oil, orange blossom, cherry blossom, cassia, caraway, cognac, jasmine, ylang-ylang, sage, fennel, piment, ginger, coriander, coffee, hemp, a mint oil from any species of the genus Mentha, star anise, cocoa, lemongrass, rooibos, flax, ginkgo biloba, hazel, hibiscus, laurel, mate, orange skin, rose, tea such as green tea or black tea, thyme, juniper, elderflower, basil, bay leaves, cumin, oregano, paprika, rosemary, saffron, lemon peel, mint, beefsteak plant, curcuma, cilantro, myrtle, cassis, valerian, pimento, mace, damien, marjoram, olive, lemon balm, lemon basil, chive, carvi, verbena, tarragon, limonene), and other additives such as charcoal, chlorophyll, minerals, botanicals, or breath freshening agents. In some embodiments, the composition comprises one or more of coffee, caramel, and mint.

Flavoring agents may be in any suitable form, for example, liquid such as an oil, solid such as a powder, or gas. In some instances, the flavoring agent may be provided in a spray-dried form or a liquid form. In some embodiments, a liquid flavorant is disposed (i.e., adsorbed or absorbed in or on) a porous particulate carrier, for example microcrystalline cellulose, which is then combined with the other composition ingredients.

The amount of flavoring agent utilized in the effervescent composition can vary, but is typically up to about 10 weight percent, and some embodiments are characterized by a flavoring agent content of at least about 0.1 weight percent, such as about 0.1 to about 10 weight percent, about 0.3 to about 5 weight percent, or about 0.5 to about 3 weight percent, based on the total dry weight of the effervescent composition.

**Sweeteners**

In order to improve the sensory properties of the effervescent composition, one or more sweeteners may be added. The sweeteners can be any sweetener or combination of sweeteners, in natural or artificial form, or as a combination of natural and artificial sweeteners. Examples of natural sweeteners include fructose, sucrose, glucose, maltose, mannose, galactose, lactose, stevia, honey, and the like. Examples of artificial sweeteners include sucralose, isomaltulose, maltodextrin, saccharin, aspartame, acesulfame K, neotame, and the like. In some embodiments, the sweetener comprises one or more sugar alcohols. Sugar alcohols are polyols derived from monosaccharides or disaccharides that have a partially or fully hydrogenated form. Sugar alcohols have, for example, about 4 to about 20 carbon atoms and include erythritol, arabitol, ribitol, isomalt, maltitol, dulcitol, iditol, mannitol, xylitol, lactitol, sorbitol, and combinations thereof (e.g., hydrogenated starch hydrolysates). In some embodiments, the sweetener is xylitol, sucralose, or a combination thereof. In some embodiments, the sweetener is sucralose.

When present, a sweetener or combination of sweeteners may make up from about 0.1 to about 20% or more of the of the effervescent composition by weight, for example, from about 0.1 to about 1%, from about 1 to about 5%, from about 5 to about 10%, or from about 10 to about 20% by weight, based on the total weight of the effervescent composition.

**Taste modifiers**

In order to improve the organoleptic properties of the effervescent composition one or more taste modifying agents ("taste modifiers") may be included which may serve to mask, alter, block, or improve e.g., the flavor of an effervescent composition as described herein. Non-limiting examples of such taste modifiers include analgesic or anesthetic herbs, spices, and flavors which produce a perceived cooling (e.g., menthol, eucalyptus, mint), warming (e.g., cinnamon), or painful (e.g., capsaicin) sensation. Certain taste modifiers fall into more than one overlapping category.

In some embodiments, the taste modifier modifies one or more of bitter, sweet, salty, or sour tastes. In some embodiments, the taste modifier targets pain receptors. In some embodiments, the effervescent composition comprises an active ingredient having a bitter taste, and a taste modifier which masks or blocks the perception of the bitter taste. In some embodiments, the taste modifier is a substance which targets pain receptors (e.g., vanilloid receptors) in the user's mouth to mask e.g., a bitter taste of another component (e.g., an active ingredient). Suitable taste modifiers include, but are not limited to, capsaicin, gamma-amino butyric acid (GABA), adenosine monophosphate (AMP), lactisole, sodium citrate,

or a combination thereof.

When present, a representative amount of taste modifier is about 0.01% by weight or more, about 0.1% by weight or more, or about 1.0% by weight or more, but will typically make up less than about 10% by weight of the total weight of the effervescent composition, (e.g., from about 0.01%, about 0.05%, about 0.1%, or about 0.5%, to about 1%, about 5%, or about 10% by weight of the total weight of the effervescent composition).

**Salts**

In some embodiments, the effervescent composition comprises a salt (e.g., an alkali metal salt), typically employed in an amount sufficient to provide desired sensory attributes to the effervescent composition. In some embodiments, certain salts may also serve as electrolytes or act in synergy with electrolytes. For example, without wishing to be bound by theory, sodium citrate may provide both a source of sodium (electrolyte) as well as aid in the absorption of other electrolytes and water. Non-limiting examples of suitable salts include sodium chloride, potassium chloride, ammonium chloride, flour salt, sodium acetate, sodium citrate, and the like. In some embodiments, the salt is sodium chloride, ammonium chloride, sodium citrate, or a combination thereof.

When present, a representative amount of salt is about 0.5% by weight or more, about 1.0% by weight or more, or about 1.5% by weight or more, but will typically make up about 10% or less of the total weight of the effervescent composition, or about 7.5% or less, or about 5% or less (e.g., from about 0.5 to about 5% by weight). In some embodiments, the effervescent composition comprises sodium citrate in an amount by weight of from about 2 to about 3%, and sodium chloride in an amount by weight of from about 0.1 to about 0.5%, based on the total weight of the effervescent composition.

**Buffering agents**

In some embodiments, the effervescent composition of the present disclosure can comprise pH adjusters or buffering agents. Examples of pH adjusters and buffering agents that can be used include, but are not limited to, metal hydroxides (e.g., alkali metal hydroxides such as sodium hydroxide and potassium hydroxide), and other alkali metal buffers such as metal carbonates (e.g., potassium carbonate or sodium carbonate), or metal bicarbonates such as sodium bicarbonate, and the like. Non-limiting examples of suitable buffers include alkali metals acetates, glycinates, phosphates, glycerophosphates, citrates, carbonates, hydrogen carbonates, borates, or mixtures thereof.

Where present, the buffering agent is typically present in an amount less than about 5% by weight, based on the weight of the effervescent composition, for example, from about 0.1% to about 5%, such as, e.g., from about 0.1% to about 1%, or from about 0.1% to about 0.5% by weight, based on the total weight of the effervescent composition.

**Colorants**

A colorant may be employed in amounts sufficient to provide the desired physical attributes to the effervescent composition. Natural or synthetic colorants, such as natural or synthetic dyes, food-grade colorants and pharmaceutical-grade colorants may be used. Examples of colorants include various dyes and pigments, such as caramel coloring and titanium dioxide. Natural colorants such as curcumin, beet juice extract, spirulina; also a variety of synthetic pigments may also be used. The amount of colorant utilized in the composition can vary, but when present is typically up to about 3% by weight, such as from about 0.1%, about 0.5%, or about 1%, to about 3% by weight, based on the total weight of the composition.

**Oral care additives**

In some embodiments, the effervescent composition comprises an oral care ingredient (or mixture of such ingredients). Oral care ingredients provide the ability to inhibit tooth decay or loss, inhibit gum disease, relieve mouth pain, whiten teeth, or otherwise inhibit tooth staining, elicit salivary stimulation, inhibit breath malodor, freshen breath, or the like. For example, effective amounts of ingredients such as thyme oil, eucalyptus oil and zinc (e.g., such as the ingredients of formulations commercially available as ZYTEX® from Discus Dental) can be incorporated into the effervescent composition. Other examples of ingredients that can be incorporated in desired effective amounts within the present effervescent composition can include those that are incorporated within the types of oral care compositions set forth in Takahashi et al., Oral Microbiology and Immunology, 19(1), 61-64 (2004); U.S. Pat. No. 6,083,527 to Thistle; and US Pat. Appl. Pub. Nos. 2006/0210488 to Jakubowski and 2006/02228308 to Cummins et al. Other example ingredients include those contained in formulations marketed as MALTISORB® by Roquette and DENTIZYME® by NatraRx. When present, a representative amount of oral care additive is at least about 1%, often at least about 3%, and frequently at least about 5% of the total dry weight of the effervescent composition. The amount of oral care additive within the effervescent composition will not typically exceed about 30%, often will not exceed about 25%, and frequently will not exceed about 20%, of the total dry

weight of the effervescent composition.

### Processing aids

If necessary for downstream processing of the effervescent composition, such as granulation, mixing, or molding, a flow aid can also be added to the effervescent composition in order to enhance flowability of the effervescent composition. Example flow aids include microcrystalline cellulose, silica, polyethylene glycol, stearic acid, calcium stearate, magnesium stearate, zinc stearate, sodium stearyl fumarate, canauba wax, and combinations thereof. In some embodiments, the flow aid is silica, stearic acid, magnesium stearate, or a combination thereof. In some embodiments, the flow aid is sodium stearyl fumarate.

When present, a representative amount of flow aid may make up at least about 0.5 percent or at least about 1 percent, of the total dry weight of the effervescent composition. Typically, the amount of flow aid within the effervescent composition will not exceed about 5 percent, and frequently will not exceed about 3 percent, of the total dry weight of the effervescent composition.

### Other additives

Other additives can be included in the disclosed effervescent composition. For example, the effervescent composition can be processed, blended, formulated, combined, and/or mixed with other materials or ingredients. The additives can be artificial or can be obtained or derived from herbal or biological sources. Examples of further types of additives include thickening or gelling agents (e.g., fish gelatin), emulsifiers, preservatives (e.g., potassium sorbate and the like), disintegration aids, or combinations thereof. See, for example, those representative components, combination of components, relative amounts of those components, and manners and methods for employing those components, set forth in US Pat. No. 9,237,769 to Mua et al., US Pat. No. 7,861,728 to Holton, Jr. et al., US Pat. App. Pub. No. 2010/0291245 to Gao et al., and US Pat. App. Pub. No. 2007/0062549 to Holton, Jr. et al., each of which is incorporated herein by reference. In some embodiments, the effervescent composition includes an emulsifier. In some embodiments, the emulsifier is lecithin.

Typical inclusion ranges for such additional additives can vary depending on the nature and function of the additive and the intended effect on the final effervescent composition, with an example range of up to about 10% by weight, based on total weight of the effervescent composition (e.g., about 0.1 to about 5% by weight).

The aforementioned additives can be employed together (e.g., as additive formulations) or separately (e.g., individual additive components can be added at different stages involved in the preparation of the final effervescent composition). Furthermore, the aforementioned types of additives may be encapsulated as provided in the final product or effervescent composition. Example encapsulated additives are described, for example, in WO2010/132444 to Atchley, which has been previously incorporated by reference herein.

### Configured for oral use

Provided herein is an effervescent composition configured for oral use. The term "configured for oral use" as used herein means that the effervescent composition is provided in a form such that during use, saliva in the mouth of the user causes the effervescent material to effervesce in the oral cavity, and saliva in the mouth of the user causes one or more of the components of the effervescent composition (e.g., flavoring agents and/or active ingredients) to pass into the mouth of the user. In some embodiments, the effervescent composition is adapted to deliver components to a user through mucous membranes in the user's mouth, the user's digestive system, or both, and, in some instances, said component is an active ingredient (including, but not limited to, for example, a stimulant) that can be absorbed through the mucous membranes in the mouth or absorbed through the digestive tract when the effervescent composition is used.

The form of the effervescent composition may vary. In some embodiments, the effervescent material is present in a granulated form. In some embodiments, the effervescent material is present in granulated form, and comprises isomalt, glycerin, and an acid and base component, each as described herein above.

In some embodiments, the effervescent material, such as the effervescent material in granulated form, is coated to enhance the properties thereof (e.g., processability, preventing moisture from prematurely initiating gas release, etc.). In some embodiments, the effervescent material is coated to minimize moisture contact with the effervescent component(s) prior to use by the consumer. Suitable coatings include cellulose ethers, shellac, gums, sugars, and lipids. By "cellulose ether" is meant a cellulose structure with the hydrogen of one or more hydroxyl groups in the cellulose polymer structure replaced with an alkyl, hydroxyalkyl, or aryl group. Cellulose ethers include, for example, alkyl ethers (e.g., methyl cellulose, ethyl cellulose), hydroxyalkyl ethers (e.g., hydroxyethyl cellulose, hydroxypropyl cellulose (HPC), hydroxyethylmethyl cellulose, hydroxypropylmethyl cellulose (HMPC), ethylhydroxyethyl cellulose), and carboxyalkyl ethers (e.g., carboxymethyl cellulose (CMC)). In some embodiments, the cellulose ether is an alkyl ether or hydroxyalkyl ether. In some

embodiments, the cellulose ether comprises one or more of methylcellulose, HPC, HPMC, hydroxyethyl cellulose, or CMC. In some embodiments, the cellulose ether is HPC.

In some embodiments, the coating comprises a lipid, such as a fat, oil, or wax substance derived from animal or plant material (e.g., plant-derived fats), typically comprising mostly triglycerides along with lesser amounts of free fatty acids and mono- or diglycerides. In some embodiments, the lipid is a solid or semi-solid at room temperature (i.e., 25°C) and capable of at least partially liquefying when subjected to the temperature of the oral cavity of the user (i.e., "melting"). Example plant-derived fats are comprised primarily of saturated or unsaturated fatty acid chains (most of which are bound within triglyceride structures) having a carbon length of about 10 to about 26 carbon atoms, or about 14 to about 20 carbon atoms, or about 14 to about 18 carbon atoms.

In some embodiments, the lipid comprises an oil and, in particular, a food grade oil. Such oils include, but are not limited to, vegetable oils (e.g., acai oil, almond oil, amaranth oil, apricot oil, apple seed oil, argan oil, avocado oil, babassu oil, beech nut oil, ben oil, bitter gourd oil, black seed oil, blackcurrant seed oil, borage seed oil, borneo tallow nut oil, bottle gourd oil, brazil nut oil, buffalo gourd oil, butternut squash seed oil, cape chestnut oil, canola oil, carob cashew oil, cocoa butter, cocklebur oil, coconut oil, corn oil, cothune oil, coriander seed oil, cottonseed oil, date seed oil, dika oil, egus seed oil, evening primrose oil, false flax oil, flaxseed oil, grape seed oil, grapefruit seed oil, hazelnut oil, hemp oil, kapok seed oil, kenaf seed oil, lallemantia oil, lemon oil, linseed oil, macadamia oil, mafura oil, marula oil, meadowfoam seed oil, mongongo nut oil, mustard oil, niger seed oil, nutmeg butter, okra seed oil, olive oil, orange oil, palm oil, palm stearin, papaya seed oil, peanut oil, pecan oil, perilla seed oil, persimmon seed oil, pequi oil, pili nut oil, pine nut oil, pistachio oil, pomegranate seed oil, poppyseed oil, pracaxi oil, prune kernel oil, pumpkin seed oil, quinoa oil, ramtil oil, rapeseed oil, rice bran oil, royle oil, sacha inchi oil, safflower oil, sapote oil, seje oil, sesame oil, shea butter, soybean oil, sunflower oil, taramira oil, tea seed oil, thistle oil, tigernut oil, tobacco seed oil, tomato seed oil, walnut oil, watermelon seed oil, wheat germ oil, and combinations thereof), animal oils (e.g., cattle fat, buffalo fat, sheep fat, goat fat, pig fat, lard, camel fat, tallow, liquid margarine, fish oil, fish liver oil, whale oil, seal oil, and combinations thereof), and mineral oils.

In some embodiments, the lipid coating includes palm oil, palm kernel oil, soybean oil, cottonseed oil, and mixtures thereof. In one embodiment, the lipid is a blend of palm oil and palm kernel oil. The lipid can be, for example, hydrogenated, partially hydrogenated, or non-hydrogenated. Example embodiments of lipids can be purchased under the brand names CEBES®, CISAO®, or CONFAO®, available from AarhusKarlshamn USA Inc, and under the brand name Paramount™, available from Bunge Loders Croklaan.

The melting point of the lipid is typically about 29°C or above, such as about 29°C to about 49°C, or about 36° C to about 45°C, or about 38°C to about 41°C. In some embodiments, use of lipids with a melting point of less than about 36° C is not advantageous due to possible melting during product storage or handling. One test for determining the melting point of lipids is the Mettler dropping point method (ASTM D3954-15, Standard Test Method for Dropping Point of Waxes, ASTM International, West Conshohocken, PA, 2015).

In some embodiments, the lipid is an oil selected from the group consisting of palm oil, palm kernel oil, soybean oil, cottonseed oil, and combinations thereof, wherein the oil may be hydrogenated, partially hydrogenated, or non-hydrogenated.

In some embodiments, the effervescent composition comprises a physical mixture of granulated effervescent material (optionally coated) and the filler component. In some embodiments, at least a portion of the active ingredient is adsorbed in or on the filler component. In some embodiments, the entirety the active ingredient is adsorbed in or on the filler component. In some embodiments, one or more additional components of the effervescent composition is adsorbed in or on the filler component, including but not limited to, sweeteners, flavoring agents, taste modifiers, salts, plasticizers, buffering agents, colorants, oral care additives, preservatives, disintegration aids, antioxidants, flow aids.

In some embodiments, the filler component comprises cellulose beads as described herein above. In some embodiments, the effervescent composition comprises a physical mixture of granulated effervescent material (optionally coated) and cellulose beads. In some embodiments, the effervescent composition is present in the pouch as a mixture of discrete particles of the effervescent material and the filler component.

The relative proportion of the filler component and granulated effervescent material in the effervescent composition may vary. In some embodiments, the effervescent composition comprises from about 40 to about 60 percent by weight of the effervescent material; and from about 40 to about 60 percent by weight of the filler component, based on the total weight of the effervescent composition. In some embodiments, the effervescent composition comprises about 40, about 45, about 50, about 55, or about 60 percent by weight of the effervescent material.

In some embodiments, the effervescent composition as described herein is disposed within a moisture-permeable container (e.g., a water-permeable pouch). Such effervescent compositions in the water-permeable pouch format are typically used by placing one pouch containing the effervescent composition in the mouth of a human subject/user. Generally, the pouch is placed somewhere in the oral cavity of the user, for example under the lips, in the same way as moist snuff products are generally used. The pouch generally is not chewed or swallowed. Exposure to saliva then causes some of the components of the effervescent composition therein (e.g., flavoring agents and/or active ingredients) to pass through e.g., the water-permeable pouch and provide the user with flavor and satisfaction, and the user is not required to

spit out any portion of the effervescent composition. After about 10 minutes to about 60 minutes, typically about 15 minutes to about 45 minutes, of use/enjoyment, substantial amounts of the effervescent composition have been absorbed through oral mucosa of the human subject, and the pouch may be removed from the mouth of the human subject for disposal.

Accordingly, in some embodiments, the effervescent composition as disclosed herein, and any other components noted above are combined within a moisture-permeable packet or pouch that acts as a container for use of the effervescent composition to provide a pouched product configured for oral use. Some embodiments of the disclosure will be described with reference to the Figure, and these described embodiments involve products having an outer pouch and containing a composition as described herein. As explained in greater detail below, such embodiments are provided by way of example only, and the pouched products of the present disclosure can include composition in other forms. The composition/construction of such packets or pouches, such as the container pouch 102 in the embodiment illustrated in the Figure, may be varied. Referring to the Figure, there is shown a first embodiment of a pouched product 100. The pouched product 100 includes a moisture-permeable container in the form of a pouch 102, which contains a material 104 comprising a composition as described herein.

The effervescent composition may be contained in pouches and packaged, in a manner and using the types of components used for the manufacture of conventional snus types of products. The pouch provides a liquid-permeable container of a type that may be considered to be similar in character to the mesh-like type of material that is used for the construction of a tea bag. Components of the effervescent composition readily diffuse through the pouch and into the mouth of the user.

Non-limiting examples of suitable types of pouches are set forth in, for example, US Pat. Nos. 5,167,244 to Kjerstad and 8,931,493 to Sebastian et al.; as well as US Patent App. Pub. Nos. 2016/0000140 to Sebastian et al.; 2016/0073689 to Sebastian et al.; 2016/0157515 to Chapman et al.; and 2016/0192703 to Sebastian et al., each of which is incorporated herein by reference. Pouches can be provided as individual pouches, or a plurality of pouches (e.g., 2, 4, 5, 10, 12, 15, 20, 25 or 30 pouches) can be connected or linked together (e.g., in an end-to-end manner) such that a single pouch or individual portion can be readily removed for use from a one-piece strand or matrix of pouches.

An example pouch may be manufactured from materials, and in such a manner, such that during use by the user, the pouch undergoes a controlled dispersion or dissolution. Such pouch materials may have the form of a mesh, screen, perforated paper, permeable fabric, or the like. For example, pouch material manufactured from a mesh-like form of rice paper, or perforated rice paper, may dissolve in the mouth of the user. As a result, the pouch and effervescent composition each may undergo complete dispersion within the mouth of the user during normal conditions of use, and hence the pouch and effervescent composition both may be ingested by the user. Other examples of pouch materials may be manufactured using water dispersible film forming materials (e.g., binding agents such as alginates, carboxymethylcellulose, xanthan gum, pullulan, and the like), as well as those materials in combination with materials such as ground cellulosics (e.g., fine particle size wood pulp). Example pouch materials, though water dispersible or dissolvable, may be designed and manufactured such that under conditions of normal use, a significant amount of the effervescent composition contents permeate through the pouch material prior to the time that the pouch undergoes loss of its physical integrity. If desired, flavoring ingredients, disintegration aids, and other desired components, may be incorporated within, or applied to, the pouch material.

A "fleece material" as used herein may be formed from various types of fibers, as described in more detail herein below, capable of being formed into a traditional fleece fabric or other traditional pouch material. For example, fleece materials may be provided in the form of a woven or nonwoven fabric. Suitable types of fleece materials are described in, for example, U.S. Patent No. 8,931,493 to Sebastian et al.; US Patent App. Pub. No. 2016/0000140 to Sebastian et al.; and US Patent App. Pub. No. 2016/0073689 to Sebastian et al.; which are all incorporated herein by reference. The term "nonwoven" is used herein in reference to fibrous materials, webs, mats, batts, or sheets in which fibers are aligned in an undefined or random orientation. The nonwoven fibers are initially presented as unbound fibers or filaments. An important step in the manufacturing of nonwovens involves binding the various fibers or filaments together. The manner in which the fibers or filaments are bound can vary, and include thermal, mechanical and chemical techniques that are selected in part based on the desired characteristics of the final product, as discussed in more detail herein below.

In some embodiments the fleece material is a nonwoven web comprising fibers and at least a portion of the fibers are selected from the group consisting of polyester fibers, tobacco-derived viscose fibers, sisal fibers, corn silk fibers, long wood fibers, MCC fibers, and combinations thereof. Such fleece materials are described further herein below, and in International Application Publication No. WO2021116853A1 to Hutchens et al., which is incorporated by reference herein in its entirety. In some embodiments, at least about 10%, at least about 20%, at least about 40%, at least about 50%, at least about 60%, at least about 80%, or at least about 90% of the fibers of the nonwoven web is selected from the group consisting of polyester fibers, viscose fibers, sisal fibers, corn silk fibers, long wood fibers, MCC fibers, and combinations thereof, based on the total weight of the fibers within the nonwoven web. In some embodiments, about 10% to about 100%, about 50% to about 100%, about 40% to about 90%, or about 60% to about 80% of the fibers of the nonwoven web is selected from the group consisting of polyester fibers, viscose fibers, sisal fibers, corn silk fibers, long wood fibers, MCC fibers, and combinations thereof, based on the total weight of the fibers within the nonwoven web. The remainder of the

fibers comprising the fleece material (where relevant) can be, in some embodiments, conventional fibrous types, including, but not limited to fibers comprising a polymer component, wool fibers, cotton fibers, conventional cellulosic fibers, and combinations thereof, as will be discussed further herein. In some embodiments, the amount of "alternative fiber" incorporated within the disclosed pouches provides some benefit to the pouch (e.g., enhanced biodegradability, enhanced mouthfeel, etc.), while not significantly negatively impacting other characteristics of the fleece (e.g., taste, strength, mouthfeel, etc.).

In some embodiments, the nonwoven web can comprise polyester fibers. As is known in the art, polyester is a category of polymer that contains ester functional groups in the main polymer chain. Polyesters include naturally occurring polymers (e.g., cutin of plant cuticles), as well as synthetically produced polymers (e.g., polybutyrate). Examples of polyesters that can be incorporated, in fiber form, within a nonwoven web pouch comprising polyester fibers include, but are not limited to, cutin, polybutyrate, polyethylene terephthalate), polyglycolide, polylactic acid, polycaprolactone, polyhydroxyalkanoate, polyhydroxybutyrate, and copolymers and derivatives thereof.

In some embodiments, the fleece material can comprise viscose fibers, including but not limited to, viscose fibers derived from tobacco. As is known in the art, viscose is a type of rayon fiber that is made from natural cellulose sources. In a typical rayon manufacturing process, cellulose is chemically converted into a soluble compound, dissolved, and then forced through a spinneret to produce filaments which are chemically solidified, resulting in fibers of regenerated cellulose. Regenerated cellulose fibers can be particularly advantageous as they can provide enhanced biodegradability and favorable sensory characteristics when used in a fleece material. Regenerated cellulose fibers are typically prepared by extracting non-cellulosic compounds from wood, contacting the extracted wood with caustic soda, followed by carbon disulfide and then by sodium hydroxide, giving a viscous solution. The solution is subsequently forced through spinneret heads to create viscous threads of regenerated fibers. Example methods for the preparation of regenerated cellulose are provided in U.S. Pat. No. 4,237,274 to Leoni et al; U.S. Pat. No. 4,268,666 to Baldini et al; U.S. Pat. No. 4,252,766 to Baldini et al.; U.S. Pat. No. 4,388,256 to Ishida et al.; U.S. Pat. No. 4,535,028 to Yokogi et al.; U.S. Pat. No. 5,441,689 to Laity; U.S. Pat. No. 5,997,790 to Vos et al.; and U.S. Pat. No. 8,177,938 to Sumnicht, which are incorporated herein by reference. The manner in which the regenerated cellulose is made is not limiting, and can include, for example, both the rayon and the TENCEL processes. Various suppliers of regenerated cellulose are known, including Lenzing (Austria), Cordenka (Germany), Aditya Birla (India), and Daicel (Japan).

In some embodiments, the fleece material can comprise at least about 10 wt. %, at least about 20 wt. %, at least about 25 wt. %, at least about 30 wt. %, at least about 40 wt. %, at least about 50 wt. %, at least about 60 wt. %, at least about 70 wt. %, at least about 80 wt. %, or at least about 90 wt. % viscose fibers, based on the total weight of the fibers within the fleece material. In some embodiments, the fleece material is 100% viscose.

In some embodiments of pouched products, the outer water-permeable pouch is made from a nonwoven web as described above. In some embodiments, the pouch is constructed of a single layer of the nonwoven web. In some embodiments, the pouch material comprises a multilayer composite made up of two or more nonwoven layers. Each nonwoven layer can be formed by processes discussed above. In a multilayer structure, the pouched product may have a first layer which can be relatively hydrophilic and a second layer which can be relatively hydrophobic (compared to each other). In some embodiments, the pouched product may comprise an outer water-permeable pouch comprising an outer hydrophilic layer and an inner hydrophobic layer in contact with the effervescent composition. As such, the hydrophobic layer can, during storage of the pouched product, retain any moisture in the composition such that flavors in the composition are not lost due to moisture loss. However, capillaries in the hydrophobic layer can wick out moisture into the mouth of the user, such that composition components are released into the oral cavity when used. In this manner, the pouch material can enhance storage stability without significantly compromising the enjoyment of the product by the end user. In some embodiments, the relatively hydrophilic layer could be located on the interior of the multi-layer structure. The two layers can be formed into a multi-layer composite nonwoven material using any means known in the art, such as by attaching the two layers together using adhesive or stitching. The hydrophobicity of a textile material can be evaluated, for example, by measuring the contact angles between a drop of liquid and the surface of a textile material, as is known in the art.

In some embodiments, an outer hydrophilic layer can comprise a flavor component (such as a flavorant as described herein), which can be applied to the nonwoven layer in any conventional manner such as by coating, printing, and the like. In some embodiments, the flavor within an outer hydrophilic layer can differ from a flavor contained within the internal composition adapted for oral use. By having a hydrophobic layer between the inner composition and the outer hydrophilic layer, the different flavors can be prevented from blending because the hydrophobic layer can prevent moisture from leaving the inner composition until enough moisture from the mouth of the user overwhelms the hydrophobic layer and thereby allows moisture to enter and leave the inner area of the pouched product where the composition is housed. By the time this takes place, the flavor component of the outer hydrophilic layer can have dissipated. In this manner, the product can be designed to provide multiple, different sensory experiences, a first sensory experience where the flavor in the outer layer transitions into the mouth of the user and a second sensory experience, typically occurring later in time, where the flavor of the internal composition transitions into the mouth of the user.

The hydrophilic and hydrophobic layers can be formed from similar nonwoven web compositions, but one of the nonwoven webs can be treated to enhance either hydrophobicity or hydrophilicity. For example, a layer of the nonwoven web can be treated with a wet chemical solution to confer hydrophilicity thereupon. In one such process, a nonwoven web layer is treated with an aqueous alcohol solution containing a food-grade surfactant. The surfactant may include, for example one or more of sorbitan aliphatic acid ester, polyglycerin aliphatic acid ester, or sucrose aliphatic acid ester (see, e.g., U.S. Pat. No. 7,498,281 to Iwasaki et al., which is incorporated herein by reference). In some embodiments, the fleece fabric layers can be made hydrophilic or hydrophobic by changing the type of fiber chosen. For example, predominantly hydrophobic cellulose fibers are commercially available as Tencel® Biosoft from Lenzing of Austria and as Olea Fiber from Kelheim of Germany. In various embodiments, the hydrophilic layer can incorporate cationic or anionic cellulose fibers that are also available from Kelheim of Germany, for example. The fibers referenced herein (polyester fibers, tobacco-derived viscose fibers, sisal fibers, corn silk fibers, long wood fibers, MCC fibers) can be incorporated in some amount within the hydrophilic layer, the hydrophobic layer, or both. The hydrophilic layer can contain additives such as polyethylene glycols, methyl cellulose, hydroxypropylmethyl cellulose, hydroxypropyl cellulose, hydroxypropylmethyl cellulose phthalate, polyvinyl pyrrolidone, polyvinyl alcohol, polyacrylic acids, gelatins, alginates, sulfosuccinates, and combinations thereof.

Generally, pouched embodiments are prepared by introducing a charge of the composition as disclosed herein to a pouch member portion by an insertion unit after a leading end of the pouch member portion has been closed, but prior to the closing of a trailing end. In some embodiments, after receiving the charge of the composition, discrete individual pouch member portions can be formed by closing the trailing end and severing the closed pouch member portion from the continuous tubular member such that an individual pouched product is formed.

Various manufacturing apparatus and methods can be used to create a pouched product described herein. For example, U.S. Patent Application Publication No. 2012/0055493 to Novak, III et al., incorporated by reference in its entirety, relates to an apparatus and process for providing pouch material formed into a tube for use in the manufacture of smokeless tobacco products. Similar apparatuses that incorporate equipment for supplying a continuous supply of a pouch material (e.g., a pouch processing unit adapted to supply a pouch material to a continuous tube forming unit for forming a continuous tubular member from the pouch material) can be used to create a pouched product described herein. Representative equipment for forming such a continuous tube of pouch material is disclosed, for example, in U.S. Patent Application Publication No. 2010/0101588 to Boldrini et al., which is incorporated herein by reference in its entirety. The apparatus further includes equipment for supplying pouched material to the continuous tubular member such that, when the continuous tubular member is subdivided and sealed into discrete pouch portions, each pouch portion includes a charge of a composition adapted for oral use. Representative equipment for supplying the filler material is disclosed, for example, in U.S. Patent Application Publication No. 2010/0018539 to Brinkley, which is incorporated herein by reference in its entirety. In some instances, the apparatus may include a subdividing unit for subdividing the continuous tubular member into individual pouch portions and, once subdivided into the individual pouch portions, may also include a sealing unit for sealing at least one of the ends of each pouch portion. In other instances, the continuous tubular member may be sealed into individual pouch portions with a sealing unit and then, once the individual pouch portions are sealed, the continuous tubular member may be subdivided into discrete individual pouch portions by a subdividing unit subdividing the continuous tubular member between the sealed ends of serially disposed pouch portions. Still in other instances, sealing (closing) of the individual pouch portions of the continuous tubular member may occur substantially concurrently with the subdivision thereof, using a closing and dividing unit. It is noted that in some embodiments of the present disclosure wherein a low melting point binder material is used, the temperature required for sealing the seams of the pouched product can be less than the temperature required in conventional processes associated with conventional binder materials.

The amount of material contained within each product unit, for example, a pouch, may vary. In some embodiments, the weight of the effervescent composition within each pouch is at least about 50 mg, for example, from about 50 mg to about 1500 mg, such as from about 100 to 800 about mg, or from about 700 to about 1500 mg. If desired, other components can be contained within each pouch. For example, at least one flavored strip, piece or sheet of flavored water dispersible or water-soluble material (e.g., a breath-freshening edible film type of material) may be disposed within each pouch along with or without at least one capsule. Such strips or sheets may be folded or crumpled in order to be readily incorporated within the pouch. See, for example, the types of materials and technologies set forth in US Pat. Nos. 6,887,307 to Scott et al. and 6,923,981 to Leung et al.; and The EFSA Journal (2004) 85, 1-32; which are incorporated herein by reference.

In some embodiments, one or more active ingredients as described herein are included in the effervescent composition within the pouched product, and optionally, one or more further active ingredients are disposed in or on the external surface of the product (e.g., on or in the pouch material as disclosed herein). The effervescent components may be incorporated in the structure of the fleece material. In some embodiments, separate location of the active ingredients may allow differential release profiles (e.g., one active ingredient may be rapidly available to the mouth and/or digestive system, and the other active ingredient may be released more gradually with product use).

A pouched product as described herein can be packaged within any suitable inner packaging material and/or outer container. See also, for example, the various types of containers for smokeless types of products that are set forth in

US Pat. Nos. 7,014,039 to Henson et al.; 7,537,110 to Kutsch et al.; 7,584,843 to Kutsch et al.; 8,397,945 to Gelardi et al., D592,956 to Thiellier; D594,154 to Patel et al.; and D625,178 to Bailey et al.; US Pat. Pub. Nos. 2008/0173317 to Robinson et al.; 2009/0014343 to Clark et al.; 2009/0014450 to Bjorkholm; 2009/0250360 to Bellamah et al.; 2009/0266837 to Gelardi et al.; 2009/0223989 to Gelardi; 2009/0230003 to Thiellier; 2010/0084424 to Gelardi; and 2010/0133140 to Bailey et al; 2010/0264157 to Bailey et al.; and 2011/0168712 to Bailey et al. which are incorporated herein by reference.

**Preparation of the effervescent composition**

The manner by which the various components of the effervescent composition (e.g., effervescent material, filler, active ingredient, and the like) are combined may vary. As such, the overall effervescent composition may be relatively uniform in nature.

In some embodiments, the effervescent material is in a granulated form. In some embodiments, the effervescent material may be prepared by mixing the various components (e.g., acid component, base component, binder, and humectant) to form a dough-like mixture. The various components of the effervescent composition may be contacted, combined, or mixed together using any mixing technique or equipment known in the art. Any mixing method that brings the effervescent composition ingredients into intimate contact can be used, such as a mixing apparatus featuring an impeller or other structure capable of agitation. Examples of mixing equipment include casing drums, conditioning cylinders or drums, liquid spray apparatus, conicaltype blenders, ribbon blenders, mixers available as FKM130, FKM600, FKM1200, FKM2000 and FKM3000 from Littleford Day, Inc., Plough Share types of mixer cylinders, Hobart mixers, and the like. See also, for example, the types of methodologies set forth in US Pat. Nos. 4,148,325 to Solomon et al.; 6,510,855 to Korte et al.; and 6,834,654 to Williams, each of which is incorporated herein by reference. In some embodiments, the components forming the effervescent composition are prepared such that the mixture thereof may be used in a starch molding process for forming the effervescent composition. Manners and methods for formulating effervescent compositions will be apparent to those skilled in the art. See, for example, the types of methodologies set forth in US Pat. No. 4,148,325 to Solomon et al.; US Pat. No. 6,510,855 to Korte et al.; and US Pat. No. 6,834,654 to Williams, US Pat. Nos. 4,725,440 to Ridgway et al., and 6,077,524 to Bolder et al., each of which is incorporated herein by reference.

The dough is then extruded. The extrusion can be carried out using extruders such as screw, sieve, basket, roll, and ram-type extruders, extruding the dough through suitably sized pierced screens. Any suitable extrudate shape may be used. In some embodiments, the dough is extruded into rods. The extrudate is then processed in a spheronizer (e.g., such as spheronizers (marumerizers) available from Caleva Process Solutions Ltd. or LCI Corporation) at a suitable rotation speed (e.g., 1200 RPM) for a suitable time (e.g., 10 minutes). For example, spheronization can be carried out using a spinning friction plate that effects rounding of extrudate particles. The variously sized granulated material (e.g., as beads) can be processed through a series of screens to provide the desired size range, such as greater than about 5, about 10, about 12, or about 16 mesh.

The effervescent material (such as the material in granular form) can include an optional outer coating as described herein above, which can help to improve storage stability of the product as well as improve the packaging process by reducing friability and dusting. In some embodiments, the effervescent material is coated to minimize moisture contact with the effervescent component(s). The coating compositions may be aqueous or oil-based in nature and can be applied using any pellet or tablet coating technique known in the art, such as pan coating.

The effervescent material (such as the material in granular form), optionally coated, may then be combined with the remaining effervescent composition components in liquid or dry solid form, and which can be admixed in a pretreatment step prior to mixture with any remaining components of the composition, or simply mixed together with all other liquid and/or dry ingredients.

In some embodiments, the effervescent material is mixed with the filler component as described herein. In some embodiments, the filler component comprises cellulosic spheres. In some embodiments, one or more ingredients of the effervescent composition are adsorbed in or on the filler component (e.g., cellulosic spheres). In some embodiments, the at least one active ingredient is adsorbed in or on the filler component. In some embodiments, a sweetener, salt, ion pairing agent, flavoring agent, or combination thereof is adsorbed in or on the filler component. Accordingly, in some embodiments, the effervescent composition is prepared by contacting one or more of the active ingredient, sweetener, salt, ion pairing agent, and flavoring agent is contacted with the filler component, either individually or as a mixture, and the filler component allowed to adsorb or adsorb the ingredient(s).

In some embodiments, the effervescent composition is enclosed in a pouch as described here in above to form a pouched product. Accordingly, in some embodiments, the product is prepared by placing the effervescent composition in a pouch and sealing the pouch as described herein above.

Many modifications and other embodiments will come to mind to one skilled in the art to which this disclosure pertains having the benefit of the teachings presented in the foregoing description. Therefore, it is to be understood that the disclosure is not to be limited to the specific embodiments disclosed and that modifications and other embodiments are intended to be included within the scope of the appended claims. Although specific terms are employed herein, they are

used in a generic and descriptive sense only and not for purposes of limitation.

## EXAMPLES

Aspects of the present disclosure are more fully illustrated by the following examples, which are set forth to illustrate certain aspects of the present disclosure and are not to be construed as limiting thereof.

### Example 1. Preparation of effervescent granules

Effervescent granules were prepared using the formulation provided in **Table 2.** The granules were prepared by mixing the isomalt, glycerin, sodium bicarbonate, citric acid, and tartaric acid together to form a homogenous blend in the form of a dough and extruding the dough. The extrudate was passed through a marumerizer to form granules, which were pan coated with 10% by weight of palm oil (Confao®5; a medium-hardness, trans-hydrogenated fat). The granulated material had a particle size distribution as shown in **Table 3.**

**Table 2:** Effervescent granule formulation

| Component | % by weight |
|---|---|
| Isomalt | 38-56 |
| Glycerin | 6-10 |
| Sodium Bicarbonate | 24 |
| Citric Acid | 14 |
| Tartaric Acid | 7 |

**Table 3.** Particle size distribution of effervescent granules

| Mesh Size | Percent Inclusion |
|---|---|
| 5 | 4.0% |
| 10 | 47.7% |
| 12 | 10.3% |
| >16 (Pass-Through) | 38.0% |

### Example 2. Preparation of cellulosic spheres

Cellulosic spheres were prepared using the formulation provided in **Table 4.** The spheres were prepared by mixing the salts, nicotine benzoate solution, sodium benzoate, sweetener, and flavoring agent with 500-micron cellulose beads.

**Table 4:** Cellulosic spheres formulation

| Component | % by weight |
|---|---|
| 500 $\mu$m Cellulose Spheres | 65-97 |
| Sodium Chloride | 2-4 |
| Ammonium Chloride | 0.1-0.3 |
| Nicotine Benzoate (25% Aqueous) | 0-12 |
| Sodium Benzoate | 0-3 |
| Sweeteners | 1-3 |
| Flavoring agent | 1-3 |

**Example 3. Preparation of pouched product with effervescent granules**

Pouched products were made by mixing the granules of Example 1 and the spheres of Example 2 together at a 1:1 ratio by weight. Portions (462 mg) of the mix were added to a fleece pouch material and sealed to form the pouched products. Effervescence was observed when pouches were placed in water.

**Example 4. Preparation of pouched product with carbonated isomalt**

Pouched products were made by mixing the carbonated isomalt (Knechtel; available from poppinfun.com) and the spheres of Example 2 together at a 1:1 ratio by weight. Portions (462 mg) of the mix were added to a fleece pouch material and sealed to form the pouched products. Effervescence was observed when pouches were placed in water.

**Example 5. Storage stability evaluation of coated effervescent materials**

Coated effervescent materials were prepared and evaluated for effervescence and taste following storage at various temperature and humidity conditions. Either carbonated isomalt crystals or the effervescent granules of Example 1 were coated with the compositions provided in **Table 5.** The coated materials were then stored for 4 days under the conditions provided in **Table 6.** Further provided in **Table 6** is the identity of the effervescent material, the coating, and the sensory evaluation results (stability and taste). "Stability" was evaluated as the sensory effervescence relative to the corresponding uncoated, freshly prepared material using five participants. Low effervescence is denoted by the symbol "-", while high effervescence is denoted by the symbol "+". Taste was similarly evaluated relative to the corresponding uncoated, freshly prepared material. A neutral taste is denoted by the symbol "++", almost neutral by the symbol "+", and an off taste by the symbol "-".

**Table 5.** Coated effervescent material compositions

| Example # | Ingredient | Weight % |
|---|---|---|
| **5A** | Carbonated Isomalt | 92.8 |
| | Shellac | 7.2 |
| **5B** | Carbonated Isomalt | 77.3 |
| | Palm oil | 16.7 |
| | Shellac | 6 |
| **5C** | Carbonated Isomalt | 68.5 |
| | Palm oil | 26.0 |
| | 30% Gum Arabic Solution | 5.5 |
| **5D** | Carbonated Isomalt | 51.4 |
| | Palm oil | 25.0 |
| | Isomalt | 19.5 |
| | 30% Gum Arabic Solution | 4.1 |
| **5E** | Carbonated Isomalt | 63.42 |
| | Isomalt | 24.1 |
| | Shellac | 7.4 |
| | 30% Gum Arabic Solution | 5.1 |
| **5F** | Carbonated Isomalt | 90.9 |
| | 5% ethyl cellulose solution | 9.1 |
| **5G** | Carbonated Isomalt | 75 |
| | Paramount X fat | 25 |
| **5H** | Carbonated Isomalt | 91.4 |
| | 30% Gum Arabic Solution | 8.6 |

(continued)

| Example # | Ingredient | Weight % |
|---|---|---|
| **5I** | Effervescent granules | 92.8 |
| | Shellac | 7.2 |
| **5J** | Effervescent granules | 77.3 |
| | Palm oil | 16.7 |
| | Shellac | 6 |
| **5K** | Effervescent granules | 68.5 |
| | Palm oil | 26.0 |
| | 30% Gum Arabic Solution | 5.5 |
| **5L** | Effervescent granules | 51.4 |
| | Palm oil | 25.0 |
| | Isomalt | 19.5 |
| | 30% Gum Arabic Solution | 4.1 |
| **5M** | Effervescent granules | 63.42 |
| | Isomalt | 24.1 |
| | Shellac | 7.4 |
| | 30% Gum Arabic Solution | 5.1 |
| **5N** | Effervescent granules | 90.9 |
| | 5% ethyl cellulose solution | 9.1 |
| **5O** | Effervescent granules | 75 |
| | Paramount X fat | 25 |

**Table 6.** Sensory evaluation of stored, coated effervescent material compositions

| Example # | Storage condition (temp., °C; relative humidity, %) | Stability | Taste |
|---|---|---|---|
| **5A** | 40C 75% RH | - | - |
| **5A** | 25C 35% RH | + | - |
| **5B** | 40C 75% RH | + | + |
| **5B** | 25C 35% RH | + | + |
| **5C** | 40C 75% RH | + | + |
| **5C** | 25C 35% RH | + | + |
| **5D** | 40C 75% RH | - | + |
| **5D** | 25C 35% RH | + | + |
| **5E** | 40C 75% RH | - | + |
| **5E** | 25C 35% RH | + | + |
| **5F** | 40C 75% RH | + | - |
| **5F** | 25C 35% RH | + | - |
| **5G** | 40C 75% RH | - | ++ |
| **5G** | 25C 35% RH | + | ++ |
| **5I** | 40C 75% RH | - | - |
| **5I** | 25C 35% RH | + | - |

(continued)

| Example # | Storage condition (temp., °C; relative humidity, %) | Stability | Taste |
|---|---|---|---|
| **5J** | 40C 75% RH | + | + |
| **5J** | 25C 35% RH | + | + |
| **5K** | 40C 75% RH | - | + |
| **5K** | 25C 35% RH | - | + |
| **5L** | 40C 75% RH | - | + |
| **5L** | 25C 35% RH | + | + |
| **5M** | 40C 75% RH | - | + |
| **5M** | 25C 35% RH | + | + |
| **5N** | 40C 75% RH | + | - |
| **5N** | 25C 35% RH | + | - |
| **5O** | 40C 75% RH | - | + |
| **5O** | 25C 35% RH | - | + |

**Claims**

**1.** A pouched product configured for oral use comprising a pouch and an effervescent composition enclosed therein, the effervescent composition comprising:

an effervescent material capable of causing effervescence in the oral cavity;
a filler component; and
at least one active ingredient.

**2.** The pouched product of claim 1, wherein the effervescent material comprises a sugar material containing an entrapped gaseous component, such that release of the entrapped gaseous component occurs upon dissolution of the sugar material in the oral cavity, such as wherein the effervescent material is carbonated isomalt.

**3.** The pouched product of claim 1, wherein the effervescent material comprises an acid component and a base component, wherein the base component is a carbonate material, a bicarbonate material, or a mixture thereof.

**4.** The pouched product of claim 3, wherein the effervescent material further comprises a binder, a humectant, or a combination thereof, optionally wherein the binder is a sugar alcohol.

**5.** The pouched product of claim 3, wherein the acid component is a tricarboxylic acid, a dicarboxylic acid, or a combination thereof, such as wherein the acid component is selected from the group consisting of citric acid, malic acid, tartaric acid, succinic acid, adipic acid, fumaric acid, gluconic acid, and combinations thereof.

**6.** The pouched product of claim 3, wherein the acid component comprises a combination of a tricarboxylic acid and a dicarboxylic acid in a weight ratio of from about 2:1 to about 1:2, such as wherein the acid component is a combination of citric acid and tartaric acid in a ratio of from about 2:1 to about 1:2 by weight.

**7.** The pouched product of claim 3, wherein the base component is a bicarbonate material.

**8.** The pouched product of claim 3, wherein the acid component is present in an amount of from about 10% to about 20% by weight, based on the total dry weight of the effervescent material and/or wherein the acid component and the base component are present in about a 1:1 molar ratio.

**9.** The pouched product of any one of claims 1 to 8, wherein the at least one active ingredient comprises one or more flavoring agents, botanical materials, stimulants, nicotine components, amino acids, vitamins, cannabinoids, cannabimimetics, terpenes, nutraceuticals, pharmaceutical agents, or combinations thereof.

**10.** The pouched product of any one of claims 1 to 8, wherein the at least one active ingredient comprises a nicotine component, such as wherein the nicotine component is selected from the group consisting of free base nicotine, a nicotine salt, an ion-paired nicotine complex, resin bound nicotine, and combinations thereof.

**11.** The pouched product of any one of claims 1 to 8, wherein the effervescent material and the filler component are present in the pouch as a mixture of discrete particles of the effervescent material and the filler component.

**12.** The pouched product of any one of claims 1 to 8, wherein the filler component comprises a cellulosic material, such as microcrystalline cellulose, optionally wherein the cellulose material is in the form of spheres.

**13.** The pouched product of any one of claims 1 to 8, wherein the effervescent material further comprises a coating, such as a coating comprising a lipid, a cellulose ether, shellac, gum arabic, or a combination thereof, optionally wherein the lipid is an oil selected from the group consisting of palm oil, palm kernel oil, soybean oil, cottonseed oil, and combinations thereof, wherein the oil may be hydrogenated, partially hydrogenated, or non-hydrogenated.

**14.** The pouched product of any one of claims 3 to 8, wherein the effervescent material is present in granulated form, the effervescent material comprising isomalt, glycerin, an acid component, and a base component, optionally wherein the isomalt is present in an amount in a range from about 38 to about 56% by weight, based on the total weight of the effervescent material, and optionally wherein the glycerin is present in an amount in a range 6 to 10 by weight, based on the total weight of the effervescent material.

**15.** The pouched product of claim 14, comprising:

from about 40 to about 60 percent by weight of the effervescent material; and
from about 40 to about 60 percent by weight of the filler component, based on the total weight of the effervescent composition.

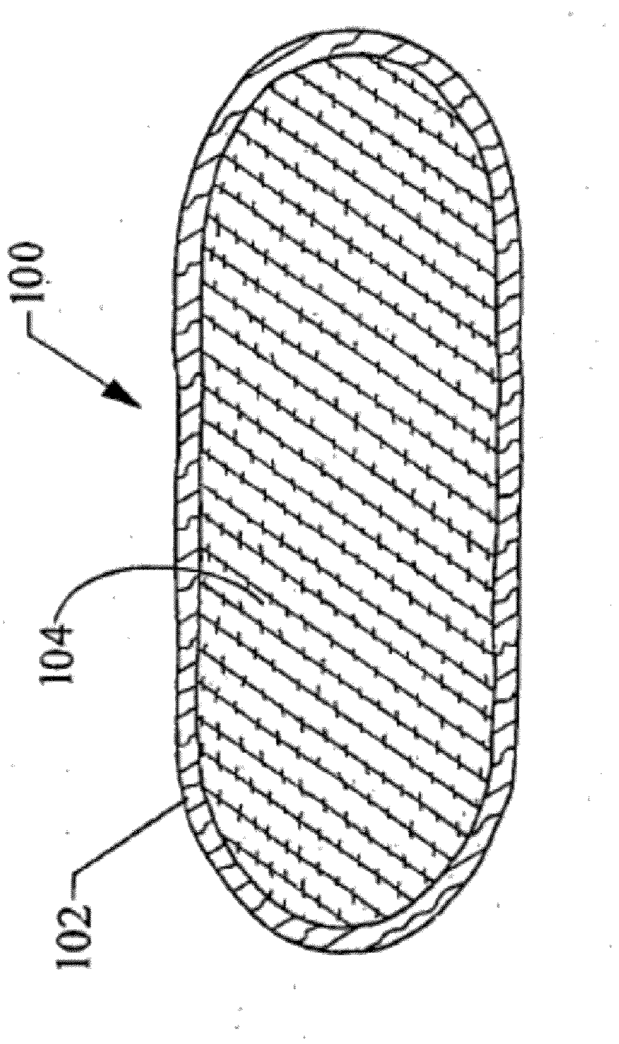


The Figure

Europäisches Patentamt
European Patent Office
Office européen des brevets

# EUROPEAN SEARCH REPORT

Application Number
EP 24 15 1724

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | US 2021/378948 A1 (GERARDI ANTHONY RICHARD [US] ET AL) 9 December 2021 (2021-12-09)<br>* page 7, paragraph [0101]; claims; examples 12, 13 *<br>* page 9, paragraph [0112] - paragraph [0114] *<br>* page 16, paragraph [0172] *<br>----- | 1-15 | INV.<br>A24B13/00<br>A24B15/16<br>A24B15/28<br>A61K9/00 |
| X | WO 2021/250516 A1 (NICOVENTURES TRADING LTD [GB]; GERARDI ANTHONY RICHARD [US]) 16 December 2021 (2021-12-16)<br>* page 33 - page 48; claims; examples 12, 13 *<br>----- | 1-15 | |
| X | US 8 424 541 B2 (CRAWFORD DANIELLE R [US]; BROOKS KAREN [US] ET AL.) 23 April 2013 (2013-04-23)<br>* column 4, line 66 - column 5, line 51 *<br>----- | 1-9,11 | |
| | | | TECHNICAL FIELDS SEARCHED (IPC) |
| | | | A24B<br>A61K |

The present search report has been drawn up for all claims

1

| Place of search | Date of completion of the search | Examiner |
|---|---|---|
| Munich | 17 May 2024 | Smeets, Dieter |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.........................................................................................................
& : member of the same patent family, corresponding document

EPO FORM 1503 03.82 (P04C01)

# ANNEX TO THE EUROPEAN SEARCH REPORT ON EUROPEAN PATENT APPLICATION NO. EP 24 15 1724

This annex lists the patent family members relating to the patent documents cited in the above-mentioned European search report. The members are as contained in the European Patent Office EDP file on
The European Patent Office is in no way liable for these particulars which are merely given for the purpose of information.

17-05-2024

| Patent document cited in search report | Publication date | Patent family member(s) | Publication date |
|---|---|---|---|
| US 2021378948 A1 | 09-12-2021 | AU 2021289162 A1 | 09-02-2023 |
| | | BR 112022024943 A2 | 27-12-2022 |
| | | CA 3181912 A1 | 16-12-2021 |
| | | EP 4161295 A1 | 12-04-2023 |
| | | JP 2023530239 A | 14-07-2023 |
| | | US 2021378948 A1 | 09-12-2021 |
| WO 2021250516 A1 | 16-12-2021 | NONE | |
| US 8424541 B2 | 23-04-2013 | US 2009025741 A1 | 29-01-2009 |
| | | US 2013236605 A1 | 12-09-2013 |
| | | WO 2009010884 A2 | 22-01-2009 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6668839 B, Williams
- US 6834654 B, Williams
- US 6953040 B, Atchley
- US 7032601 B, Atchley
- US 7694686 B, Atchley
- US 7810507 B, Dube
- US 7819124 B, Strickland
- US 7861728 B, Holton, Jr.
- US 7901512 B, Quinter
- US 8627828 B, Strickland
- US 11246334 B, Atchley
- US 20080196730, Engstrom
- US 20080305216 A, Crawford
- US 20090293889 A, Kumar
- US 20100291245 A, Gao
- US 20110139164 A, Mua
- US 20120037175 A, Cantrell
- US 20120055494 A, Hunt
- US 20120138073 A, Cantrell
- US 20120138074 A, Cantrell
- US 20130074855 A, Holton, Jr.
- US 20130074856 A, Holton, Jr.
- US 20130152953 A, Mua
- US 20130274296 A, Jackson
- US 20150068545 A, Moldoveanu
- US 20150101627 A, Marshall
- US 20150230515 A, Lampe
- US 20220160675, Gerardi
- US 20220071984 A, Poole
- US 20210378948 A, Gerardi
- US 20210330590 A, Hutchens
- US 20210186081 A, Gerardi
- US 20210177754 A, Keller
- US 20210177043 A, Gerardi
- US 20210177038 A, Gerardi
- US 20210169867 A, Holton, Jr.
- US 20210169792 A, Holton, Jr.
- US 20210169132 A, Holton, Jr.
- US 20210169121 A, St. Charles
- US 20210169122 A, St. Charles
- US 4289794 A, Kleiner
- US 5165951 A, Gallart
- US 5439698 A, Ahn
- US 4639368 A, Niazi
- US 5178878 A, Wehling
- US 5223264 A, Wehling
- US 6974590 B, Pather
- US 7381667 B, Bergquist
- US 20060191548 A, Strickland
- US 20090025741 A, Crawford
- US 20100018539 A, Brinkley
- US 20100170522 A, Sun
- WO 9706786 A, Johnson
- US 20040191322 A, Hansson
- US 2033909 A, Cox
- US 3901248 A, Lichtneckert
- US 7189760 B, Erman
- US 6592884 B, Hofmann
- US 4230688 A
- US 4032661 A
- US 4459425 A
- US 4178459 A
- US 4296255 A
- US 4136163 A
- US 5009893 A
- US 5266592 A
- US 5698181 A
- US 6277385 B
- US 6627233 B
- US 7030273 B
- US 20050222256 A
- US 20050265930 A
- US 6780443 B
- US 6159509 A
- US 5545424 A
- US 5407665 A
- US 6083527 A, Thistle
- US 20060210488, Jakubowski
- US 200602228308, Cummins
- US 9237769 B, Mua
- US 20100291245, Gao
- US 20070062549, Holton, Jr.
- WO 2010132444 A, Atchley
- US 5167244 A, Kjerstad
- US 8931493 A, Sebastian
- US 20160000140, Sebastian
- US 20160073689, Sebastian
- US 20160157515, Chapman
- US 20160192703, Sebastian
- US 8931493 B, Sebastian
- WO 2021116853 A1, Hutchens
- US 4237274 A, Leoni
- US 4268666 A, Baldini
- US 4252766 A, Baldini
- US 4388256 A, Ishida
- US 4535028 A, Yokogi
- US 5441689 A, Laity
- US 5997790 A, Vos

- US 8177938 B, Sumnicht
- US 7498281 B, Iwasaki
- US 20120055493, Novak, III
- US 20100101588, Boldrini
- US 20100018539, Brinkley
- US 6887307 B, Scott
- US 6923981 B, Leung
- US 7014039 B, Henson
- US 7537110 B, Kutsch
- US 7584843 B, Kutsch
- US 8397945 B, Gelardi
- US D592956 B, Thiellier
- US D594154 B, Patel
- US D625178 B, Bailey
- US 20080173317 A, Robinson
- US 20090014343 A, Clark
- US 20090014450 A, Bjorkholm
- US 20090250360 A, Bellamah
- US 20090266837 A, Gelardi
- US 20090223989 A, Gelardi
- US 20090230003 A, Thiellier
- US 20100084424 A, Gelardi
- US 20100133140 A, Bailey
- US 20100264157 A, Bailey
- US 20110168712 A, Bailey
- US 4148325 A, Solomon
- US 6510855 A, Korte
- US 6834654 A, Williams
- US 6510855 B, Korte
- US 4725440 A, Ridgway
- US 6077524 A, Bolder

**Non-patent literature cited in the description**


- **PERFETTI**. *Beitrage Tabakforschung Int.*, 1983, vol. 12, 43-54
- **TAKAHASHI et al.** *Oral Microbiology and Immunology*, 2004, vol. 19 (1), 61-64
- *The EFSA Journal*, 2004, vol. 85, 1-32